# EUROPEAN PATENT APPLICATION

(11) **EP 1 586 648 A1**
(43) Date of publication of application: **19.10.2005**
(21) Application number: 03701795.1
(22) Date of filing: 20.01.2003
(51) Int. Cl.: C12N 15/54, C12N 9/10, C12N 1/15, C12N 1/19, C12N 1/21, C12N 5/10, C07K 16/40, C12Q 1/02, C12Q 1/68, G01N 33/53, A01N 63/02, A01K 67/00

(54) **JUVENILE HORMONE TRANSMETHYLASE GENES AND METHOD OF USING THE SAME**

(71) Applicant: Incorporated Administrative Agency, National Agriculture and Bio-Oriented Research Organization, Tsukuba-shi, Ibaraki 305-8517 (JP)
(72) Inventor: SHINODA, Tetsuro, Ano-cho, Age-gun, Mie 514-2323 (JP); ITOYAMA, Kyo, Tsu-shi, Mie 514-0102 (JP); HAMAMURA, Tetsuzo, Tsu-shi, Mie 514-0125 (JP)
(74) Representative: Kalhammer, Georg
(86) International application number: PCT/JP2003/000415
(87) International publication number: WO 2004/065604

(57) **Abstract**

Juvenile hormone acid methyltransferase gene was cloned using a cDNA derived from the corpora allata of *Bombyx mori* by differential display methods. A recombinant protein expressed in *Escherichia coli,* which was transformed with a vector DNA incorporating the gene, was found to have juvenile hormone acid methyltransferase activity. Based on amino acid sequence homology, juvenile hormone acid methyltransferase genes were found from *Drosophila melanogaster, Anopheles gambiae, Spodoptera litura,* and *Helicoverpa armigera.* The proteins encoded by the juvenile hormone acid methyltransferase genes derived from *Drosophila melanogaster, Spodoptera litura,* and *Helicoverpa armigera* were found to have juvenile hormone acid methyltransferase activities.

SEQUENCE LISTING

<110>  National Agricultural Research Organization

<120>  JUVENILE HORMONE TRANSMETHYLASE GENES
       AND METHOD OF USING THE SAME

<130>  ARO-A0301P

<160>  36

<170>  PatentIn version 3.1

<210>  1
<211>  2890
<212>  DNA
<213>  Bombyx mori

<220>
<221>  CDS
<222>  (125)..(961)
<223>

<400>  1
   aaaagactgt tcggcgatac cgcagtgaga acagcgacac atcgcacgcg cttagttcaa      60
   tacgaactta aaaaaaaaaa gtgccgacaa atattttatt gcaaaaaaaa atattaagtc 120
   aaaa atg aac aat gca gat tta tac cgc aaa agt aac agc ctc caa aag 169
   Met Asn Asn Ala Asp Leu Tyr Arg Lys Ser Asn Ser Leu Gln Lys
   1 5 10 15
   aga gat gct ctg cgc tgc ctc gag gaa cat gcg aat aag att aag tgg 217
   Arg Asp Ala Leu Arg Cys Leu Glu Glu His Ala Asn Lys Ile Lys Trp
   20 25 30
   aaa aaa atc ggt gat aga gtc ata gat ttg ggt tgc gct gac ggt agt 265
   Lys Lys Ile Gly Asp Arg Val Ile Asp Leu Gly Cys Ala Asp Gly Ser
   35 40 45
   gtt act gat att ttg aaa gtt tac atg cca aaa aat tac gga aga tta 313
   Val Thr Asp Ile Leu Lys Val Tyr Met Pro Lys Asn Tyr Gly Arg Leu
   50 55 60
   gtt ggc tgc gac ata agc gaa gaa atg gtg aaa tac gcg aat aaa cac 361
   Val Gly Cys Asp Ile Ser Glu Glu Met Val Lys Tyr Ala Asn Lys His
   65 70 75
   cac ggc ttc ggt agg act tcg ttc agg gtg ctc gac ata gag ggc gat 409
   His Gly Phe Gly Arg Thr Ser Phe Arg Val Leu Asp Ile Glu Gly Asp
   80 85 90 95
   ttg acc gca gac ctg aaa caa ggc ttt gat cac gtt ttc tct ttt tac 457
   Leu Thr Ala Asp Leu Lys Gln Gly Phe Asp His Val Phe Ser Phe Tyr
   100 105 110
   acc ctc cac tgg atc aga gac cag gaa cga gct ttc cgg aac att ttc 505
   Thr Leu His Trp Ile Arg Asp Gln Glu Arg Ala Phe Arg Asn Ile Phe
   115 120 125
   aat ctg ctt ggc gac gaa gga gat tgc tta ctt ctg ttc ctg ggc cac 553
   Asn Leu Leu Gly Asp Glu Gly Asp Cys Leu Leu Leu Phe Leu Gly His
   130 135 140
   act cct atc ttt gat gtt tac cgc aca ctc tcg cat aca gaa aaa tgg 601
   Thr Pro Ile Phe Asp Val Tyr Arg Thr Leu Ser His Thr Glu Lys Trp
   145 150 155
   cat tct tgg ctc gag cac gtc gat cga ttt ata tca cct tac cat gac 649
   His Ser Trp Leu Glu His Val Asp Arg Phe Ile Ser Pro Tyr His Asp
   160 165 170 175
   aat gaa gat ccc gaa aaa gaa gtt aag aag ata atg gaa aga gtt gga 697
   Asn Glu Asp Pro Glu Lys Glu Val Lys Lys Ile Met Glu Arg Val Gly
   180 185 190
   ttt agc aat att gaa gtg caa tgc aaa acg ttg ttc tat gta tac gat 745
   Phe Ser Asn Ile Glu Val Gln Cys Lys Thr Leu Phe Tyr Val Tyr Asp
   195 200 205
   gat ttg gat gtt tta aaa aaa tca gta gca gct atc aat cca ttt aat 793
   Asp Leu Asp Val Leu Lys Lys Ser Val Ala Ala Ile Asn Pro Phe Asn
   210 215 220
   atc ccg aaa gac ata ctt gaa gac ttt tta gaa gat tac ata gat gta 841
   Ile Pro Lys Asp Ile Leu Glu Asp Phe Leu Glu Asp Tyr Ile Asp Val
   225 230 235
   gta cgg gag atg agg ctg ctc gac agg tgt aat aac aat gtc ggt gaa 889
   Val Arg Glu Met Arg Leu Leu Asp Arg Cys Asn Asn Asn Val Gly Glu
   240 245 250 255
   tca gtg tct ata aaa ttt aac tac aaa gtc atc agt gtt tac gcg aga 937
   Ser Val Ser Ile Lys Phe Asn Tyr Lys Val Ile Ser Val Tyr Ala Arg
   260 265 270
   aaa tta tgt tta agt tta atg taa ttaagttaga aaaaaaaaac aaatccgact 991
   Lys Leu Cys Leu Ser Leu Met
   275
   tctaaatctt ggaaataagt ttttttaaca tatttctctc tctttgtctt cccagatttt 1051
   cgtgattaag ctttctattc actattttaa aaagctttct caatatattg aatacagttt 1111
   agaatacagt tggtatctga aatcactgat ttcgaagtcg gttacttaga acaaatttaa 1171
   ttagttaatt gtatttataa ggaacgctac aatatttatt acagtaagta attttaaatt 1231
   tccggaaaca gtggagttca gtggacaatg cagtatcagg agatggctta gatggatgga 1291
   cgagctcaca gtcacccggt gttaagtggt tactggagcc catagacatc tacaatgtaa 1351
   atgccgccac cgatatgagt tataaggtct cagaatagct gccccaccct tcaagccgaa 1411
   acgcgttact gcttcacggc agaaataggc agggcggtgg tacctacccg cgcggactta 1471
   caagagctcc taccactagt gagagccagc tgctttttgt tgtcgtgtaa aatcggtcgt 1531
   tgtctctagt catatcatag ccgtacaaaa aaatagacaa cttgatatta taactgttct 1591
   tcaaatttct cgtcacacat acgaaaataa agtttgatgt atttacccga aatataaatg 1651
   ttaacatagc tcatacatac atacatatat aattcatata tacacattaa gttttaatac 1711
   tctgaaatat gtctacccca aaacaagaaa gcctactgta ctaaaattgt atgcagtttt 1771
   agttttaatg tattatttta ataacagtga catctattag cgatacattg aactatttca 1831
   acacaacatt tttcatatta ttttcctttg aatctaatta ctttttttaa gccgcagtaa 1891
   gatggcggtg ttgtacgact taaagtgttg agaatttaaa ataatttcta cggttgttaa 1951
   attataattg aaatagaaat ccatactatt ttaattactc aactcaacaa ctcaattatt 2011
   ttttaaagga gatgtgtgag tgagcatcac tagagaattg tttgaccgtt gaccgattgg 2071
   gctgatttct ttttcattat atccgtaaat tttaggacaa gttttgtacc accaactaca 2131
   gccactacag tattcattga tttttaatta ccataagttt ggtttgtgta aagtatattt 2191
   ataataacga taaatttggt ttgagtcgtg atagttggac atttcaacta gtccgcttgc 2251
   tcatttgatt atactatgtg cttagtgcgg gttcttttac gctatcgaga acgttaaaga 2311
   actcgcacta agcacactat tgtattttat ttgttgcaca aatatattta gaaacaatcg 2371
   tgtataatca tcatgcttta aaagtattcc tataaaatca taaacagtca caaaatttat 2431
   ttttagtgag aattacatga aacaaattat gaaacaaagt ttgattatac tttcaaatgt 2491
   ttcattactg tttttaagct taaggtataa aatggcatgt ttgtgtgttt actcaggccc 2551
   taagcaacac gcgttggctt ttaagtaaac tttgaagtct tagttattcc tgggtttatt 2611
   tggcacgtta gtaaaatcta ccctgccatt tagtaccatt agagtgcgaa tgattacagc 2671
   gtatgattat tgtttacatt tttaattttt gttaattact taacgaaacc tgcccgggat 2731
   ttattgacaa cataattagt aaaacatgtg ataatttgaa caatgacaaa tttattgtta 2791
   gttaatttat ttcactttgt atataataat tgtaagtgtg atgtttaaat aaataaaact 2851
   aaagtgtaaa aaaaaaaaaa aaaaaaaaaa aaaaaaaaa 2890

<210> 2

<211> 278

<212> PRT

<213> Bombyx mori

<400> 2

Met Asn Asn Ala Asp Leu Tyr Arg Lys Ser Asn Ser Leu Gln Lys Arg

1               5                   10                  15

Asp Ala Leu Arg Cys Leu Glu Glu His Ala Asn Lys Ile Lys Trp Lys

            20                  25                  30

Lys Ile Gly Asp Arg Val Ile Asp Leu Gly Cys Ala Asp Gly Ser Val

        35                  40                  45

Thr Asp Ile Leu Lys Val Tyr Met Pro Lys Asn Tyr Gly Arg Leu Val

    50                  55                  60

Gly Cys Asp Ile Ser Glu Glu Met Val Lys Tyr Ala Asn Lys His His
65                  70                  75                  80

Gly Phe Gly Arg Thr Ser Phe Arg Val Leu Asp Ile Glu Gly Asp Leu
                85                  90                  95

Thr Ala Asp Leu Lys Gln Gly Phe Asp His Val Phe Ser Phe Tyr Thr
            100                 105                 110

Leu His Trp Ile Arg Asp Gln Glu Arg Ala Phe Arg Asn Ile Phe Asn
        115                 120                 125

Leu Leu Gly Asp Glu Gly Asp Cys Leu Leu Leu Phe Leu Gly His Thr
    130                 135                 140

Pro Ile Phe Asp Val Tyr Arg Thr Leu Ser His Thr Glu Lys Trp His
145                 150                 155                 160

Ser Trp Leu Glu His Val Asp Arg Phe Ile Ser Pro Tyr His Asp Asn
                165                 170                 175

Glu Asp Pro Glu Lys Glu Val Lys Lys Ile Met Glu Arg Val Gly Phe
            180                 185                 190

Ser Asn Ile Glu Val Gln Cys Lys Thr Leu Phe Tyr Val Tyr Asp Asp

195                 200                 205

Leu Asp Val Leu Lys Lys Ser Val Ala Ala Ile Asn Pro Phe Asn Ile

    210                 215                 220

Pro Lys Asp Ile Leu Glu Asp Phe Leu Glu Asp Tyr Ile Asp Val Val

225                 230                 235                 240

Arg Glu Met Arg Leu Leu Asp Arg Cys Asn Asn Asn Val Gly Glu Ser

                245                 250                 255

Val Ser Ile Lys Phe Asn Tyr Lys Val Ile Ser Val Tyr Ala Arg Lys

            260                 265                 270

Leu Cys Leu Ser Leu Met

        275

<210>  3

<211>  938

<212>  DNA

<213>  Drosophila melanogaster

<220>

<221>  CDS

<222>  (1)..(894)

<223>

<400> 3

atg aat cag gcc tct cta tat cag cac gcc aac cag gtg caa agg cac 48

Met Asn Gln Ala Ser Leu Tyr Gln His Ala Asn Gln Val Gln Arg His

1               5                   10                  15

gat gcc aaa ttg att ctg gat gag ttc gca tcg acc atg cag tgg cga 96

Asp Ala Lys Leu Ile Leu Asp Glu Phe Ala Ser Thr Met Gln Trp Arg

            20                  25                  30

tcc gat gga gag gat gcc ctc ctg gat gtg ggt tca gga tct ggt aac 144

Ser Asp Gly Glu Asp Ala Leu Leu Asp Val Gly Ser Gly Ser Gly Asn

        35                  40                  45

gtg ctc atg gac ttt gta aag cca ctg ctc ccg att cgt gga caa ctg 192

Val Leu Met Asp Phe Val Lys Pro Leu Leu Pro Ile Arg Gly Gln Leu

    50                  55                  60

gtg ggc aca gac atc tcc agc cag atg gtg cac tat gcc agt aag cat 240

Val Gly Thr Asp Ile Ser Ser Gln Met Val His Tyr Ala Ser Lys His

65                  70                  75                  80

tac cag cga gag gag cgt acc aga ttc cag gtt ctg gac att gga tgc 288

Tyr Gln Arg Glu Glu Arg Thr Arg Phe Gln Val Leu Asp Ile Gly Cys

                85                  90                  95

gaa cgg ctg ccc gag gag ctg agc ggc aga ttt gac cat gtc acc tcg 336
Glu Arg Leu Pro Glu Glu Leu Ser Gly Arg Phe Asp His Val Thr Ser
            100                 105                 110

ttc tac tgc ctc cat tgg gtg caa aat ctg aaa gga gct ctc gga aat 384
Phe Tyr Cys Leu His Trp Val Gln Asn Leu Lys Gly Ala Leu Gly Asn
        115                 120                 125

atc tac aat ctt ctg aag ccc gaa ggt ggc gac tgc ctc ctg gca ttt 432
Ile Tyr Asn Leu Leu Lys Pro Glu Gly Gly Asp Cys Leu Leu Ala Phe
    130                 135                 140

ttg gcc tcc aat ccg gtt tac gaa gtc tat aag att ctt aaa acg aat 480
Leu Ala Ser Asn Pro Val Tyr Glu Val Tyr Lys Ile Leu Lys Thr Asn
145                 150                 155                 160

gac aag tgg tcg act tat atg cag gat gtg gag aac ttc ata tcc cca 528
Asp Lys Trp Ser Thr Tyr Met Gln Asp Val Glu Asn Phe Ile Ser Pro
                165                 170                 175

ctt cac tac agt cta aat cct ggc gag gaa ttc agc cag ttg ttg aac 576
Leu His Tyr Ser Leu Asn Pro Gly Glu Glu Phe Ser Gln Leu Leu Asn
            180                 185                 190

gat gtg ggt ttc gtg caa cac aat gtg gaa att cga aac gaa gtg ttt 624

Asp Val Gly Phe Val Gln His Asn Val Glu Ile Arg Asn Glu Val Phe
        195                 200                 205

gtt tat gaa ggt gta agg act ctg aaa gat aat gta aag gcc att tgt 672
Val Tyr Glu Gly Val Arg Thr Leu Lys Asp Asn Val Lys Ala Ile Cys
    210                 215                 220

cct ttt ctt gag cga atg cct gca gat ttg cat gaa cag ttc ctg gat 720
Pro Phe Leu Glu Arg Met Pro Ala Asp Leu His Glu Gln Phe Leu Asp
225                 230                 235                 240

gac ttc ata gac att gtt ata tcc atg aat ttg cag caa ggt gaa aat 768
Asp Phe Ile Asp Ile Val Ile Ser Met Asn Leu Gln Gln Gly Glu Asn
                245                 250                 255

aat gag gat caa aag ttc cta tct ccc tat aaa ctg gtg gtg gcc tat 816
Asn Glu Asp Gln Lys Phe Leu Ser Pro Tyr Lys Leu Val Val Ala Tyr
            260                 265                 270

gct cgc aag act cct gaa ttt gtg aat aat gtt ttc ctg gag cct aca 864
Ala Arg Lys Thr Pro Glu Phe Val Asn Asn Val Phe Leu Glu Pro Thr
        275                 280                 285

cat caa aac ttg gtt aag gga ata aat taa ttttatttta caaattaaca 914
His Gln Asn Leu Val Lys Gly Ile Asn
    290                 295

gtaattgcat ttgttaacag agtc 938

<210> 4

<211> 297

<212> PRT

<213> Drosophila melanogaster

<400> 4

Met Asn Gln Ala Ser Leu Tyr Gln His Ala Asn Gln Val Gln Arg His

1               5                   10                  15

Asp Ala Lys Leu Ile Leu Asp Glu Phe Ala Ser Thr Met Gln Trp Arg

            20                  25                  30

Ser Asp Gly Glu Asp Ala Leu Leu Asp Val Gly Ser Gly Ser Gly Asn

        35                  40                  45

Val Leu Met Asp Phe Val Lys Pro Leu Leu Pro Ile Arg Gly Gln Leu

    50                  55                  60

Val Gly Thr Asp Ile Ser Ser Gln Met Val His Tyr Ala Ser Lys His

65                  70                  75                  80

Tyr Gln Arg Glu Glu Arg Thr Arg Phe Gln Val Leu Asp Ile Gly Cys

                85                  90                  95

Glu Arg Leu Pro Glu Glu Leu Ser Gly Arg Phe Asp His Val Thr Ser

            100                 105                 110

Phe Tyr Cys Leu His Trp Val Gln Asn Leu Lys Gly Ala Leu Gly Asn

        115                 120                 125

Ile Tyr Asn Leu Leu Lys Pro Glu Gly Gly Asp Cys Leu Leu Ala Phe

    130                 135                 140

Leu Ala Ser Asn Pro Val Tyr Glu Val Tyr Lys Ile Leu Lys Thr Asn

145                 150                 155                 160

Asp Lys Trp Ser Thr Tyr Met Gln Asp Val Glu Asn Phe Ile Ser Pro

                165                 170                 175

Leu His Tyr Ser Leu Asn Pro Gly Glu Glu Phe Ser Gln Leu Leu Asn

            180                 185                 190

Asp Val Gly Phe Val Gln His Asn Val Glu Ile Arg Asn Glu Val Phe

        195                 200                 205

Val Tyr Glu Gly Val Arg Thr Leu Lys Asp Asn Val Lys Ala Ile Cys

    210                 215                 220

Pro Phe Leu Glu Arg Met Pro Ala Asp Leu His Glu Gln Phe Leu Asp
225                 230                 235                 240

Asp Phe Ile Asp Ile Val Ile Ser Met Asn Leu Gln Gln Gly Glu Asn
                245                 250                 255

Asn Glu Asp Gln Lys Phe Leu Ser Pro Tyr Lys Leu Val Val Ala Tyr
            260                 265                 270

Ala Arg Lys Thr Pro Glu Phe Val Asn Asn Val Phe Leu Glu Pro Thr
        275                 280                 285

His Gln Asn Leu Val Lys Gly Ile Asn
    290                 295

<210> 5
<211> 855
<212> DNA
<213> Anopheles gambiae

<220>
<221> CDS
<222> (1)..(855)
<223>

<400> 5

atg aac aaa gct aat ctc tac cag caa gcg aat ggc gtg cag cgg cgc 48

Met Asn Lys Ala Asn Leu Tyr Gln Gln Ala Asn Gly Val Gln Arg Arg

1               5                   10                  15

gat gcg atg gag ata ttg aaa gaa tac gca cca ctc atc agc tgt cca 96

Asp Ala Met Glu Ile Leu Lys Glu Tyr Ala Pro Leu Ile Ser Cys Pro

            20                  25                  30

agg gat ggc gaa gag gtt gcc ctg tta gac att ggg tgt ggc agc ggg 144

Arg Asp Gly Glu Glu Val Ala Leu Leu Asp Ile Gly Cys Gly Ser Gly

        35                  40                  45

gac gtg ttg gtg gac tac att cta ccg gtg ctg agc cgc gga agc act 192

Asp Val Leu Val Asp Tyr Ile Leu Pro Val Leu Ser Arg Gly Ser Thr

    50                  55                  60

cct gtg gcg cgc gca ttg gcg aca gac atc tcg gag cag atg gtg cgc 240

Pro Val Ala Arg Ala Leu Ala Thr Asp Ile Ser Glu Gln Met Val Arg

65                  70                  75                  80

cat gca cgt gaa tcg tac cgt cac gtt aag acg atc gag ttc gac acc 288

His Ala Arg Glu Ser Tyr Arg His Val Lys Thr Ile Glu Phe Asp Thr

                85                  90                  95

ctt gac atc ggc atc aaa ctc gat agt gca aag ttg tcc cgc tgg gga 336

Leu Asp Ile Gly Ile Lys Leu Asp Ser Ala Lys Leu Ser Arg Trp Gly
            100                 105                 110

caa ttt agt cac gtt acc tcg ttc tac tgc cta cac tgg gtt cag aat 384
Gln Phe Ser His Val Thr Ser Phe Tyr Cys Leu His Trp Val Gln Asn
        115                 120                 125

cag cac gta gcc ttt tcc aac atc tac aac ctt ctg cag cat ggc ggt 432
Gln His Val Ala Phe Ser Asn Ile Tyr Asn Leu Leu Gln His Gly Gly
    130                 135                 140

gac tgt ctg ctg gtg ttt ttg gca aac aac cca atc ttt gac ata tac 480
Asp Cys Leu Leu Val Phe Leu Ala Asn Asn Pro Ile Phe Asp Ile Tyr
145                 150                 155                 160

aac caa ctg tct cgc tca ccc aag tgg tcc aag tat atg tac gac gtg 528
Asn Gln Leu Ser Arg Ser Pro Lys Trp Ser Lys Tyr Met Tyr Asp Val
                165                 170                 175

gaa aag tac att tcg ccc tac cag tat tgt gaa aat cca gca agt gag 576
Glu Lys Tyr Ile Ser Pro Tyr Gln Tyr Cys Glu Asn Pro Ala Ser Glu
            180                 185                 190

atc gag gat ctg ctg tgc acg gtg ggc ttt cag cag tat cag ata cag 624
Ile Glu Asp Leu Leu Cys Thr Val Gly Phe Gln Gln Tyr Gln Ile Gln
        195                 200                 205

gtt cgc gac aag ctg tac gtg tac gaa ggg ctg gac aat ttg aaa cga 672
Val Arg Asp Lys Leu Tyr Val Tyr Glu Gly Leu Asp Asn Leu Lys Arg
    210                 215                 220

gca gtt ttg gct gtg aat cca ttc agt gaa cga atg cca cca gaa ctg 720
Ala Val Leu Ala Val Asn Pro Phe Ser Glu Arg Met Pro Pro Glu Leu
225                 230                 235                 240

cag gat agg ttc cta ctg gac tat atc gct gtt gtg cgg cag atg tac 768
Gln Asp Arg Phe Leu Leu Asp Tyr Ile Ala Val Val Arg Gln Met Tyr
                245                 250                 255

ttg acc aaa act ggc agc gaa gag aat gat tgc aat ctt caa ttc ata 816
Leu Thr Lys Thr Gly Ser Glu Glu Asn Asp Cys Asn Leu Gln Phe Ile
            260                 265                 270

tcc cca tac aag ttg gtg gtt gtt tat gcg aag aaa taa 855
Ser Pro Tyr Lys Leu Val Val Val Tyr Ala Lys Lys
        275                 280

<210> 6
<211> 284
<212> PRT
<213> Anopheles gambiae

<400> 6

Met Asn Lys Ala Asn Leu Tyr Gln Gln Ala Asn Gly Val Gln Arg Arg

1               5                   10                  15

Asp Ala Met Glu Ile Leu Lys Glu Tyr Ala Pro Leu Ile Ser Cys Pro

            20                  25                  30

Arg Asp Gly Glu Glu Val Ala Leu Leu Asp Ile Gly Cys Gly Ser Gly

        35                  40                  45

Asp Val Leu Val Asp Tyr Ile Leu Pro Val Leu Ser Arg Gly Ser Thr

    50                  55                  60

Pro Val Ala Arg Ala Leu Ala Thr Asp Ile Ser Glu Gln Met Val Arg

65                  70                  75                  80

His Ala Arg Glu Ser Tyr Arg His Val Lys Thr Ile Glu Phe Asp Thr

                85                  90                  95

Leu Asp Ile Gly Ile Lys Leu Asp Ser Ala Lys Leu Ser Arg Trp Gly

            100                 105                 110

Gln Phe Ser His Val Thr Ser Phe Tyr Cys Leu His Trp Val Gln Asn

        115                 120                 125

Gln His Val Ala Phe Ser Asn Ile Tyr Asn Leu Leu Gln His Gly Gly
    130                 135                 140

Asp Cys Leu Leu Val Phe Leu Ala Asn Asn Pro Ile Phe Asp Ile Tyr
145                 150                 155                 160

Asn Gln Leu Ser Arg Ser Pro Lys Trp Ser Lys Tyr Met Tyr Asp Val
                165                 170                 175

Glu Lys Tyr Ile Ser Pro Tyr Gln Tyr Cys Glu Asn Pro Ala Ser Glu
            180                 185                 190

Ile Glu Asp Leu Leu Cys Thr Val Gly Phe Gln Gln Tyr Gln Ile Gln
        195                 200                 205

Val Arg Asp Lys Leu Tyr Val Tyr Glu Gly Leu Asp Asn Leu Lys Arg
    210                 215                 220

Ala Val Leu Ala Val Asn Pro Phe Ser Glu Arg Met Pro Pro Glu Leu
225                 230                 235                 240

Gln Asp Arg Phe Leu Leu Asp Tyr Ile Ala Val Val Arg Gln Met Tyr
                245                 250                 255

Leu Thr Lys Thr Gly Ser Glu Glu Asn Asp Cys Asn Leu Gln Phe Ile
            260                 265                 270

Ser Pro Tyr Lys Leu Val Val Val Tyr Ala Lys Lys
        275                 280

<210>  7
<211>  994
<212>  DNA
<213>  Spodoptera litura

<220>
<221>  CDS
<222>  (111)..(932)
<223>

<400>  7
agcgactgtc cgccgcaacc gcgctccact caaccacgcg tacgcgcacc cgaaaaaaaa      60

caatacaaaa aatcttttcg agttaactga aaatcttaaa aaaatcaacc atg aat      116
                                                       Met Asn
                                                       1

aac gcc gtt ttg tac gaa caa gct aac agc atg cag aag aga gat gca      164
Asn Ala Val Leu Tyr Glu Gln Ala Asn Ser Met Gln Lys Arg Asp Ala
        5                   10                  15

ctc aac ttt tta gac gac atc tcc cct aaa cta aag tgg aag aag agt 212
Leu Asn Phe Leu Asp Asp Ile Ser Pro Lys Leu Lys Trp Lys Lys Ser
    20                  25                  30

atc agc aac att ctg gac gta gga tgt gga gat gga tgt gtc acc agc 260
Ile Ser Asn Ile Leu Asp Val Gly Cys Gly Asp Gly Cys Val Thr Ser
35                  40                  45                  50

atg ctt aaa aag tac atc cct act gac ttc aag ctg ctc ggc tgt gac 308
Met Leu Lys Lys Tyr Ile Pro Thr Asp Phe Lys Leu Leu Gly Cys Asp
                55                  60                  65

atc agt gag aag atg gtg aat ttc gcc aat gac cac cat tgc aat gaa 356
Ile Ser Glu Lys Met Val Asn Phe Ala Asn Asp His His Cys Asn Glu
            70                  75                  80

cag acg tcg ttc aca gtg ctg gac atc gca gga gat ata ccc gaa ggt 404
Gln Thr Ser Phe Thr Val Leu Asp Ile Ala Gly Asp Ile Pro Glu Gly
        85                  90                  95

atg aag gga aaa ttc gac cat gtt ttc tcc ttc tat gcc ttg cac tgg 452
Met Lys Gly Lys Phe Asp His Val Phe Ser Phe Tyr Ala Leu His Trp
    100                 105                 110

gtc ttg gat caa gag cgc gta ttc agg aat att tac gat ttg ctg agt 500
Val Leu Asp Gln Glu Arg Val Phe Arg Asn Ile Tyr Asp Leu Leu Ser

115                 120                 125                 130

aaa gat gga gaa tgc ttc acg ata ttt gtc gca ggc gca ccc gtg ttc     548
Lys Asp Gly Glu Cys Phe Thr Ile Phe Val Ala Gly Ala Pro Val Phe
                135                 140                 145

gac ttg tac cgc att tta tcg cgt aac aac aaa tgg agc act ctg ctt     596
Asp Leu Tyr Arg Ile Leu Ser Arg Asn Asn Lys Trp Ser Thr Leu Leu
            150                 155                 160

aaa gat gtc gag aaa tac ata tcg cca tac cac gac tca cag gat cca     644
Lys Asp Val Glu Lys Tyr Ile Ser Pro Tyr His Asp Ser Gln Asp Pro
        165                 170                 175

gcg aaa gaa atg aga aaa gta ttg gaa aaa gtt gga tac gtg gac tac     692
Ala Lys Glu Met Arg Lys Val Leu Glu Lys Val Gly Tyr Val Asp Tyr
    180                 185                 190

aag gtg gaa tgt aaa aac ttg gtg tat atg tac aac aac ttc gcc agt     740
Lys Val Glu Cys Lys Asn Leu Val Tyr Met Tyr Asn Asn Phe Ala Ser
195                 200                 205                 210

tta tgg aaa acc ctt caa gca atc aac cca ttc aac atc ccg aaa gat     788
Leu Trp Lys Thr Leu Gln Ala Ile Asn Pro Phe Asn Ile Pro Lys Asp
                215                 220                 225

atg gaa gaa gat ttc aaa caa gat tac tta aat att tta aaa gat atg 836
Met Glu Glu Asp Phe Lys Gln Asp Tyr Leu Asn Ile Leu Lys Asp Met
            230                 235                 240

aaa att gtg tct aag tat aat acc gat gag gca agt gtg aac ttc aaa 884
Lys Ile Val Ser Lys Tyr Asn Thr Asp Glu Ala Ser Val Asn Phe Lys
        245                 250                 255

tat cgg ttg ctt gtc gta cac gct cgc aag ccg gcc tca gaa ttt tag 932
Tyr Arg Leu Leu Val Val His Ala Arg Lys Pro Ala Ser Glu Phe
    260                 265                 270

ggaaaataat cacaaataaa ctaactgaat atttgtagta caataacaaa acctgatgca 992

ag 994

<210> 8
<211> 273
<212> PRT
<213> Spodoptera litura

<400> 8
Met Asn Asn Ala Val Leu Tyr Glu Gln Ala Asn Ser Met Gln Lys Arg
1               5                   10                  15

Asp Ala Leu Asn Phe Leu Asp Asp Ile Ser Pro Lys Leu Lys Trp Lys
            20                  25                  30
Lys Ser Ile Ser Asn Ile Leu Asp Val Gly Cys Gly Asp Gly Cys Val
        35                  40                  45
Thr Ser Met Leu Lys Lys Tyr Ile Pro Thr Asp Phe Lys Leu Leu Gly
    50                  55                  60
Cys Asp Ile Ser Glu Lys Met Val Asn Phe Ala Asn Asp His His Cys
65                  70                  75                  80
Asn Glu Gln Thr Ser Phe Thr Val Leu Asp Ile Ala Gly Asp Ile Pro
                85                  90                  95
Glu Gly Met Lys Gly Lys Phe Asp His Val Phe Ser Phe Tyr Ala Leu
            100                 105                 110
His Trp Val Leu Asp Gln Glu Arg Val Phe Arg Asn Ile Tyr Asp Leu
        115                 120                 125
Leu Ser Lys Asp Gly Glu Cys Phe Thr Ile Phe Val Ala Gly Ala Pro
    130                 135                 140
Val Phe Asp Leu Tyr Arg Ile Leu Ser Arg Asn Asn Lys Trp Ser Thr
145                 150                 155                 160

Leu Leu Lys Asp Val Glu Lys Tyr Ile Ser Pro Tyr His Asp Ser Gln

                165                 170                 175

Asp Pro Ala Lys Glu Met Arg Lys Val Leu Glu Lys Val Gly Tyr Val

            180                 185                 190

Asp Tyr Lys Val Glu Cys Lys Asn Leu Val Tyr Met Tyr Asn Asn Phe

        195                 200                 205

Ala Ser Leu Trp Lys Thr Leu Gln Ala Ile Asn Pro Phe Asn Ile Pro

    210                 215                 220

Lys Asp Met Glu Glu Asp Phe Lys Gln Asp Tyr Leu Asn Ile Leu Lys

225                 230                 235                 240

Asp Met Lys Ile Val Ser Lys Tyr Asn Thr Asp Glu Ala Ser Val Asn

                245                 250                 255

Phe Lys Tyr Arg Leu Leu Val Val His Ala Arg Lys Pro Ala Ser Glu

            260                 265                 270

Phe

<210> 9

<211> 1193

<212> DNA

<213> Helicoverpa armigera

<220>

<221> CDS

<222> (124)..(978)

<223>

<400> 9
   actgtccgcc gaaaccgcgc ttcactcaac cacgcgcacg cgcacccgaa aataaataac 60
   aagaatcata aactaaacaa atcaatatca cgaaattcaa atacctataa aaaaatcctt 120
   aaa atg aat aac gcg gtc ttg tat gaa aaa agc aat agc ttg cag aag 168
   Met Asn Asn Ala Val Leu Tyr Glu Lys Ser Asn Ser Leu Gln Lys
   1 5 10 15
   aga gat gct atc atg tgt cta gaa gaa tac gct tcg aaa att aag tgg 216
   Arg Asp Ala Ile Met Cys Leu Glu Glu Tyr Ala Ser Lys Ile Lys Trp
   20 25 30
   aag aag agt aat aat aat att ctt gac ata ggc tgt ggg gat gga agc 264
   Lys Lys Ser Asn Asn Asn Ile Leu Asp Ile Gly Cys Gly Asp Gly Ser
   35 40 45
   gtg act aat atg ctg aag aaa tac atc cct act gag tac aag ttg ctt 312
   Val Thr Asn Met Leu Lys Lys Tyr Ile Pro Thr Glu Tyr Lys Leu Leu
   50 55 60
   ggc tgt gat att agc gag aag atg gtg aac ttc gcg aat gat cat cat 360
   Gly Cys Asp Ile Ser Glu Lys Met Val Asn Phe Ala Asn Asp His His
   65 70 75
   tgt aac gaa cag act tct ttc acc gtg ctc gat att gag gga gac cta 408
   Cys Asn Glu Gln Thr Ser Phe Thr Val Leu Asp Ile Glu Gly Asp Leu
   80 85 90 95
   cct gaa ggt atg aag gga aac ttc gac cac gtt ttc tcg ttc tac gct 456
   Pro Glu Gly Met Lys Gly Asn Phe Asp His Val Phe Ser Phe Tyr Ala
   100 105 110
   ctg cac tgg gtt aat aac caa gaa cga gca ttc aaa aac ata tac aac 504
   Leu His Trp Val Asn Asn Gln Glu Arg Ala Phe Lys Asn Ile Tyr Asn
   115 120 125
   ctt cta agc gag gat ggg gag tgc ttc acg ata ttc gta gcc tgg gct 552
   Leu Leu Ser Glu Asp Gly Glu Cys Phe Thr Ile Phe Val Ala Trp Ala
   130 135 140
   cct gtg ttt gac gtg tac cga gtg ctc gcg cgc aac aac aag tgg agt 600
   Pro Val Phe Asp Val Tyr Arg Val Leu Ala Arg Asn Asn Lys Trp Ser
   145 150 155
   caa tgg gtg cat gat gtc gac aga tac ata tcg ccc tac cac gac tct 648
   Gln Trp Val His Asp Val Asp Arg Tyr Ile Ser Pro Tyr His Asp Ser
   160 165 170 175
   ttg gag ccg gaa aaa gat tta aag gct atg ata gac aaa att gga ttc 696
   Leu Glu Pro Glu Lys Asp Leu Lys Ala Met Ile Asp Lys Ile Gly Phe
   180 185 190
   gtt gac atc gat gtg gaa tgt aaa gaa ttg gta ttc gtg tac gac aac 744
   Val Asp Ile Asp Val Glu Cys Lys Glu Leu Val Phe Val Tyr Asp Asn
   195 200 205
   ata cat att ttg cga aaa gcg tta aca gca atc aac cct ttc aaa atc 792
   Ile His Ile Leu Arg Lys Ala Leu Thr Ala Ile Asn Pro Phe Lys Ile
   210 215 220
   ccc aag gaa aaa tat gat gat ttc atg gaa gac tat atg gat ata ctg 840
   Pro Lys Glu Lys Tyr Asp Asp Phe Met Glu Asp Tyr Met Asp Ile Leu
   225 230 235
   aaa gaa cta caa att tta gac aag tac aac aat aat tat gaa aag agc 888
   Lys Glu Leu Gln Ile Leu Asp Lys Tyr Asn Asn Asn Tyr Glu Lys Ser
   240 245 250 255
   gtt gaa ttc aat tac cgt ttg ctt gta gtg tat gcc cga aaa cct gac 936
   Val Glu Phe Asn Tyr Arg Leu Leu Val Val Tyr Ala Arg Lys Pro Asp
   260 265 270
   tcg cag gat aaa atg tta gaa gct cta aat gga caa acg tag 978
   Ser Gln Asp Lys Met Leu Glu Ala Leu Asn Gly Gln Thr
   275 280
   actgaaaaac ttatattttt agttacggca aaatacagtg tagaacagtt atttgtagtt 1038
   aaggatgaat gtatagtgta tctcttcagg tttagttttg ggcctggtat gaaatgttgt 1098
   ttttttaagt aagctatttt ggtaatgtaa actattttta aaggcaggaa aataatctgt 1158
   gtgtgagcaa aaaaaaaaaa aaaaaaaaaa aaaaa 1193

<210> 10
<211> 284
<212> PRT
<213> Helicoverpa armigera

<400> 10
Met Asn Asn Ala Val Leu Tyr Glu Lys Ser Asn Ser Leu Gln Lys Arg
1               5                   10                  15

Asp Ala Ile Met Cys Leu Glu Glu Tyr Ala Ser Lys Ile Lys Trp Lys
            20                  25                  30
Lys Ser Asn Asn Asn Ile Leu Asp Ile Gly Cys Gly Asp Gly Ser Val
        35                  40                  45
Thr Asn Met Leu Lys Lys Tyr Ile Pro Thr Glu Tyr Lys Leu Leu Gly
    50                  55                  60
Cys Asp Ile Ser Glu Lys Met Val Asn Phe Ala Asn Asp His His Cys
65                  70                  75                  80
Asn Glu Gln Thr Ser Phe Thr Val Leu Asp Ile Glu Gly Asp Leu Pro
                85                  90                  95
Glu Gly Met Lys Gly Asn Phe Asp His Val Phe Ser Phe Tyr Ala Leu
            100                 105                 110
His Trp Val Asn Asn Gln Glu Arg Ala Phe Lys Asn Ile Tyr Asn Leu
        115                 120                 125
Leu Ser Glu Asp Gly Glu Cys Phe Thr Ile Phe Val Ala Trp Ala Pro
    130                 135                 140
Val Phe Asp Val Tyr Arg Val Leu Ala Arg Asn Asn Lys Trp Ser Gln
145                 150                 155                 160

Trp Val His Asp Val Asp Arg Tyr Ile Ser Pro Tyr His Asp Ser Leu
                165                 170                 175
Glu Pro Glu Lys Asp Leu Lys Ala Met Ile Asp Lys Ile Gly Phe Val
            180                 185                 190
Asp Ile Asp Val Glu Cys Lys Glu Leu Val Phe Val Tyr Asp Asn Ile
        195                 200                 205
His Ile Leu Arg Lys Ala Leu Thr Ala Ile Asn Pro Phe Lys Ile Pro
    210                 215                 220
Lys Glu Lys Tyr Asp Asp Phe Met Glu Asp Tyr Met Asp Ile Leu Lys
225                 230                 235                 240
Glu Leu Gln Ile Leu Asp Lys Tyr Asn Asn Asn Tyr Glu Lys Ser Val
                245                 250                 255
Glu Phe Asn Tyr Arg Leu Leu Val Val Tyr Ala Arg Lys Pro Asp Ser
            260                 265                 270
Gln Asp Lys Met Leu Glu Ala Leu Asn Gly Gln Thr
        275                 280

<210> 11

<211> 17

<212> DNA

<213> Artificial

<220>

<223> an artificially synthesized primer sequence

<400> 11

tttttttttt tttttgc 17

<210> 12

<211> 10

<212> DNA

<213> Artificial

<220>

<223> an artificially synthesized primer sequence

<400> 12

gatcatagcc 10

<210> 13

<211> 25

<212>  DNA
<213>  Artificial

<220>
<223>  an artificially synthesized primer sequence

<400>  13
aagccgcagt aagatggcgg tgttg                                          25

<210>  14
<211>  25
<212>  DNA
<213>  Artificial

<220>
<223>  an artificially synthesized primer sequence

<400>  14
caacaccgcc atcttactgc ggctt                                          25

<210>  15
<211>  30
<212>  DNA
<213>  Artificial

<220>

<223>  an artificially synthesized primer sequence

<400>  15
aaacatatga acaatgcaga tttataccgc                                        30

<210>  16
<211>  30
<212>  DNA
<213>  Artificial

<220>

<223>  an artificially synthesized primer sequence

<400>  16
aaggatccaa tcacgaaaat ctgggaagac                                        30

<210>  17
<211>  30
<212>  DNA
<213>  Artificial

<220>

<223>  an artificially synthesized primer sequence

<400>  17
aaacatatga atcaggcctc tctatatcag                                        30

<210>  18
<211>  34
<212>  DNA
<213>  Artificial

<220>
<223>  an artificially synthesized primer sequence

<400>  18
aaggatccga ctctgttaac aaatgcaatt actg                                   34

<210>  19
<211>  23
<212>  DNA
<213>  Artificial

<220>
<223>  an artificially synthesized primer sequence

<220>

<221> misc_feature

<222> (6)..(6)

<223> "n"=A,T,G or C

<220>

<221> misc_feature

<222> (15)..(15)

<223> "n"=A,T,G or C

<400> 19

atggtnaart aygcnaayaa rca 23

<210> 20

<211> 23

<212> DNA

<213> Artificial

<220>

<223> an artificially synthesized primer sequence

<220>

<221> misc_feature

<222> (6)..(6)

<223> "n"=A,T,G or C

<220>

<221>  misc_feature

<222>  (12)..(12)

<223>  "n"=A,T,G or C


<400>  20

taraangara anacrtgrtc raa                                              23




<210>  21

<211>  28

<212>  DNA

<213>  Artificial


<220>

<223>  an artificially synthesized primer sequence


<400>  21

ttcacagtgc tggacatcgc aggagata                                         28




<210>  22

<211>  28

<212>  DNA

<213>  Artificial

<220>

<223>  an artificially synthesized primer sequence

<400>  22
tatctcctgc gatgtccagc actgtgaa                                          28

<210>  23
<211>  26
<212>  DNA
<213>  Artificial

<220>

<223>  an artificially synthesized primer sequence

<400>  23
cgaacagact tctttcaccg tgctcg                                            26

<210>  24
<211>  26
<212>  DNA
<213>  Artificial

<220>

<223>  an artificially synthesized primer sequence

<400>  24
cgagcacggt gaaagaagtc tgttcg                                          26

<210>  25
<211>  30
<212>  DNA
<213>  Artificial

<220>
<223>  an artificially synthesized primer sequence

<400>  25
aaacatatga ataacgccgt tttgtacgaa                                      30

<210>  26
<211>  30
<212>  DNA
<213>  Artificial

<220>
<223>  an artificially synthesized primer sequence

<400> 26

aactcgagct tgcatcaggt tttgttattg 30

<210> 27

<211> 30

<212> DNA

<213> Artificial

<220>

<223> an artificially synthesized primer sequence

<400> 27

aaacatatga ataacgcggt cttgtatgaa 30

<210> 28

<211> 30

<212> DNA

<213> Artificial

<220>

<223> an artificially synthesized primer sequence

<400> 28

aactcgaggc ccaaaactaa acctgaagag 30

<210>  29

<211>  8

<212>  PRT

<213>  Artificial

<220>

<223>  consensus sequence

<220>

<221>  MISC_FEATURE

<222>  (2)..(2)

<223>  "Xaa"=Asp or Glu

<220>

<221>  MISC_FEATURE

<222>  (3)..(3)

<223>  "Xaa"=hydrophobic amino acid

<220>

<221>  MISC_FEATURE

<222>  (5)..(5)

<223>  "Xaa"=low molecular weight neutral amino acid

<220>

<221>  MISC_FEATURE
<222>  (7)..(7)
<223>  "Xaa"=low molecular weight neutral amino acid

<400>  29
Leu Xaa Xaa Gly Xaa Gly Xaa Gly
1               5

<210>  30
<211>  18
<212>  PRT
<213>  Artificial

<220>
<223>  consensus sequence

<220>
<221>  MISC_FEATURE
<222>  (5)..(5)
<223>  "Xaa"=any amino acid

<220>
<221>  MISC_FEATURE
<222>  (8)..(8)
<223>  "Xaa"=Gln or Glu

<220>

<221> MISC_FEATURE

<222> (9)..(9)

<223> "Xaa"=His, Lys or Gln

<220>

<221> MISC_FEATURE

<222> (13)..(13)

<223> "Xaa"=hydrophobic amino acid

<400> 30

Met Asn Asn Ala Xaa Leu Tyr Xaa Xaa Ala Asn Ser Xaa Gln Lys Arg

1               5                   10                  15

Asp Ala

<210> 31

<211> 9

<212> PRT

<213> Artificial

<220>

<223> consensus sequence

<220>

<221> MISC_FEATURE

<222> (1)..(1)

<223> "Xaa"=Leu, Val or Ile

<220>

<221> MISC_FEATURE

<222> (4)..(4)

<223> "Xaa"=Leu, Val or Ile

<400> 31

Xaa Leu Asp Xaa Gly Cys Gly Asp Gly

1               5

<210> 32

<211> 12

<212> PRT

<213> Artificial

<220>

<223> consensus sequence

<220>

<221> MISC_FEATURE

<222>  (1)..(1)

<223>  "Xaa"=Gln, Arg or Lys

<220>

<221>  MISC_FEATURE

<222>  (3)..(3)

<223>  "Xaa"=Leu or Val

<220>

<221>  MISC_FEATURE

<222>  (10)..(10)

<223>  "Xaa"=Gln or Lys

<400>  32

Xaa Leu Xaa Gly Cys Asp Ile Ser Glu Xaa Met Val

1               5                   10

<210>  33

<211>  13

<212>  PRT

<213>  Artificial

<220>

<223>  consensus sequence

<220>

<221> MISC_FEATURE

<222> (9)..(9)

<223> "Xaa"=Cys, Thr or Ala

<400> 33

Phe Asp His Val Phe Ser Phe Tyr Xaa Leu His Trp Val

1               5                   10

<210> 34

<211> 9

<212> PRT

<213> Artificial

<220>

<223> consensus sequence

<220>

<221> MISC_FEATURE

<222> (2)..(2)

<223> "Xaa"=Val or Ile

<220>

<221> MISC_FEATURE

<222> (5)..(5)

<223>  "Xaa"=Val, Ile or Leu

<220>
<221>  MISC_FEATURE
<222>  (8)..(8)
<223>  "Xaa"=Ile or Val

<400>  34
Pro Xaa Phe Asp Xaa Tyr Arg Xaa Leu
1               5

<210>  35
<211>  12
<212>  PRT
<213>  Artificial

<220>
<223>  consensus sequence

<220>
<221>  MISC_FEATURE
<222>  (4)..(4)
<223>  "Xaa"=Lys or Arg

<400>  35

Asp Val Glu Xaa Tyr Ile Ser Pro Tyr His Asp Ser
1               5                   10

<210>  36
<211>  10
<212>  PRT
<213>  Artificial

<220>
<223>  consensus sequence

<220>
<221>  MISC_FEATURE
<222>  (4)..(4)
<223>  "Xaa"=Ile, Val or Leu

<400>  36
Tyr Lys Leu Xaa Val Val Tyr Ala Arg Lys
1               5                   10

## Description

### Technical Field

The present invention relates to juvenile hormone acid methyltransferase proteins, DNAs encoding the proteins, and methods of using the same.

### Background Art

Juvenile hormone is an insect hormone with a specific sesquiterpene structure, which is involved in regulation of many physiological phenomena of insects, including not only molting/metamorphosis, reproduction, diapause, and embryonic development, but also polymorphism, behavior, and life span (Non-patent Documents 1 and 2).

Therefore, it is expected that agricultural/forest pests or medically significant pests may be controlled by disturbing the juvenile hormone balance within an insect's body, thus disturbing the physiological functions essential for its survival (Non-patent Documents 3 and 4). Many synthetic juvenile hormone active substances exhibiting stronger activities than natural juvenile hormones have been synthesized, and some have been already put to practical use as extremely safe pest control agents (Non-patent Documents 5 and 6).

In addition, by adequately controlling the amount of juvenile hormone in insects, the growth and diapause of beneficial insects, such as silkworms, can be controlled, and these functions can be enhanced. For example, methoprene, a synthetic juvenile hormone active substance, is used as a cocoon-promoting agent for silkworms (Non-patent Document 7).

However, since juvenile hormone active substances do not function during the larval stage, when there is a high concentration of juvenile hormone within the insect body, a strong effect in preventing insect feeding damage cannot be expected for many agricultural pests with a damage-causing larval stage.

As a better means for control, crop damage may be reduced by blocking juvenile hormone activity or by decreasing the concentration of juvenile hormone in the insect body, thus shortening the feeding term of a larva, or promoting precocious metamorphism. So far, twenty or more types of compounds such as Precocene have been identified as anti-juvenile hormone substances, but no compound has been put into practical use for insect control (Non-patent Document 8). Attempts have been made to artificially reduce juvenile hormone concentration in the blood using genetic engineering techniques. First, an insect virus incorporating the gene for juvenile hormone esterase, which specifically hydrolyzes juvenile hormone in the blood, is produced. Then the enzyme activity is expressed in an insect body infected with the virus, to degrade juvenile hormone *in vivo* (Non-patent Document 9). However, even this method is not yet effective for precocious metamorphism of a larva.

Juvenile hormones are synthesized in the corpora allata, an endocrine organ characteristic of insects, and then secreted in the blood. Accordingly, if juvenile hormone synthesis is directly inhibited in the corpora allata, the hormones in an insect body may be reduced.

In the last stage of juvenile hormone biosynthesis, juvenile hormone methyltransferase transfers the methyl group of S-adenosyl methionine into juvenile hormone acid (an inactive precursor) and produces active juvenile hormone (Non-patent Document 10). This enzyme activity decreases in final instar larvae, which stops the synthesis and secretion of juvenile hormone. The juvenile hormone then disappears from blood, which initiates metamorphosis. Therefore, by artificially regulating this enzyme activity, it is thought the amount of juvenile hormone in an insect body can be changed to an arbitrary level, enabling pest control and enhanced functioning for beneficial insects.

Because juvenile hormone acid methyltransferase is present in minute amounts in the corpora allata, which is a minute tissue, it is very difficult to prepare amounts of protein necessary to screen compounds that bind to the enzyme, and compounds which control the activity or expression of the enzyme. Further, the amino acid sequence and the nucleotide sequence of the gene encoding the enzyme have not been revealed until now.

Documents of related prior arts for the present invention are described below.
[Non-patent Document 1] Nijhout, H.F.: Insect hormones, Princeton University Press, 267pp, 1994
[Non-patent Document 2] Herman, W. & Tatar, M.: Proc. R. Soc. Lond. B Biol. Sci. 268:2509, 2001
[Non-patent Document 3] Cusson, M & Palli, S.R.: Can. Entomol. 132:263, 2000
[Non-patent Document 4] Hiroshi Fukami: Seibutsu ni Manabu Nouyaku no Sousei (Creation of herbicides in view of living organisms), SOFT SCIENCE. Inc., p.19-38, 1986
[Non-patent Document 5] Morifusa Eto: Seibutsu ni Manabu Nouyaku no Sousei (Creation of herbicides in view of living organisms), SOFT SCIENCE. Inc., p.1-18, 1986
[Non-patent Document 6] Hatakoshi et al.: Sumitomo Chemical Co., Ltd., p.4-20, 1997-I
[Non-patent Document 7] Hiroshi Akai: Seiri Kassei busshitsu no Bioassay (Bioassay of Physiologically Active Substances), Kodansha Ltd., p.383-388, 1984
[Non-patent Document 8] Hiroshi Fukami: Seibutsu ni Manabu Nouyaku no Sousei (Creation of herbicides in view of living organisms), SOFT SCIENCE Ltd., p.19-38, 1986
[Non-patent Document 9] Hammock, B.D. et al.: US patent, 5098706, 1992
[Non-patent Document 10] Schooley, D.A. & Baker, F.C.: Comp. Insect Physiol. Biochem. Pharmacol., Pergammon Press, Oxford, 7:368-379, 1985

### Disclosure of the Invention

The present invention was accomplished in view of the above-mentioned situation. An object of the invention is to provide juvenile hormone acid methyltransferase proteins, DNAs encoding the proteins, and methods of using them.

To solve the above problem, the present inventors focused on the fact that juvenile hormone acid methyltransferase in the corpora allata of a Lepidoptera insect maintains a high activity until the penultimate instar larvae, but that activity disappears after the early stage of the final instar larva. The present inventors employed differential display methods to clone a juvenile hormone acid methyltransferase gene, using cDNAs derived from the corpora allata of *Bombyx mori.* They acquired vector DNAs incorporating this gene, and then found that recombinant proteins produced with *Escherichia coli,* genetically transformed with this vector DNA, had juvenile hormone acid methyltransferase activity.

Furthermore, based on amino acid sequence homologies, the present inventors derived juvenile hormone acid methyltransferase genes from *Drosophila melanogaster, Anopheles gambiae, Spodoptera litura,* and *Helicoverpa armigera.* They also acquired vector DNAs incorporated with the juvenile hormone acid methyltransferase genes derived from *Drosophila melanogaster, Spodoptera litura,* and *Helicoverpa armigera,* and found that recombinant proteins produced with *Escherichia coli* transformed with these vector DNAs had juvenile hormone acid methyltransferase activity. Thus, it became possible to artificially control expression of juvenile hormone acid methyltransferase proteins.

Juvenile hormones are involved in the regulation of many physiological phenomena of insects, such as molting/metamorphosis, reproduction, diapause, blastogenesis, behavior, polymorphism, and life span. Therefore, by artificially controlling the expression of juvenile hormone methyltransferase proteins, it is possible to regulate the molting/metamorphosis, reproduction, diapause, blastogenesis, behavior, polymorphism and life span of insects. For example, juvenile hormone methyltransferase proteins can be artificially regulated to control pests, or to enhance the functions of beneficial insects. Further, juvenile hormone methyltransferase proteins can be used to produce or screen for compounds that bind them, and compounds that regulate their activity or expression. The compounds obtained by screening can be used as highly safe growth regulatory agents that act specifically on insects.

Namely, the present invention relates to juvenile hormone acid methyltransferase proteins, DNAs encoding the proteins, and methods of using them, and provides [1] to [22] described below:
[1] A DNA encoding a protein having a juvenile hormone acid methyltransferase activity, according to any one of (a) to (d) below:
   (a) a DNA encoding a protein comprising an amino acid sequence of SEQ ID NO: 2, 4, 6, 8, or 10;
   (b) a DNA comprising a coding region for a nucleotide sequence of SEQ ID NO: 1, 3, 5, 7, or 9;
   (c) a DNA encoding a protein comprising an amino acid sequence of SEQ ID NO: 2, 4, 6, 8, or 10, wherein one or more amino acids are substituted, deleted, inserted, and/or added; or
   (d) a DNA that hybridizes under stringent conditions with a DNA comprising a nucleotide sequence of SEQ ID NO: 1, 3, 5, 7, or 9.
[2] A protein encoded by the DNA of [1].
[3] A DNA according to any one of (a) to (c) below:
   (a) a DNA encoding an antisense RNA complementary to a transcription product of a DNA of [1];
   (b) a DNA encoding an RNA having a ribozyme activity which specifically cleaves a transcription product of a DNA of [1]; or
   (c) a DNA encoding an RNA which inhibits the expression of a DNA of [1] by an RNAi effect.
[4] A vector inserted with the DNA of [1] or [3].
[5] An agent for regulating molting/metamorphosis, reproduction, diapause, blastogenesis, behavior, polymorphism, or life span of an arthropod, comprising as an active ingredient the DNA of [1], or a vector inserted with said DNA.
[6] The agent of [5], wherein the arthropod is an insect.
[7] The agent of [6], wherein the regulatory agent is a reproductive maturation accelerating agent, a diapause terminating agent, or a life span shortening agent for an adult insect, a metamorphosis inhibiting agent for a larva and a pupa, or a diapause inducing agent for a larva.
[8] The agent of [6], wherein the regulatory agent is a pest control agent or a cocoon-promoting agent.
[9] An agent for controlling molting/metamorphosis, reproduction, diapause, blastogenesis, behavior, polymorphism, or life span of an arthropod, comprising as an active ingredient the DNA of [3], or a vector inserted with said DNA.
[10] The agent of [9], wherein the arthropod is an insect.
[11] The agent of [10], wherein the regulatory agent is a reproductive maturation inhibiting agent, a diapause inducing agent, or a life span elongating agent for an adult insect, a diapause inhibiting agent or a metamorphosis inducing agent for a larva.
[12] The agent of [10], wherein the regulatory agent is a pest control agent.
[13] A transformed cell retaining the DNA of [1] or [3], or a vector of [4].
[14] An individual transformed with the DNA of [1] or [3], or a vector of [4].
[15] The individual of [14], wherein the individual is an insect.
[16] An antibody that binds to the protein of [2].
[17] The antibody of [16], wherein the antibody is a monoclonal antibody.
[18] An oligonucleotide comprising at least 15 nucleotides, wherein the oligonucleotide is complementary to the DNA of [1] or its complementary strand.
[19] A method of screening for a compound that binds to the protein of [2], comprising steps
   (a) to (c) below:

   (a) contacting a test compound with said protein;
   (b) detecting the binding of the test compound and said protein; and
   (c) selecting a compound which binds to said protein.
[20] A method of screening for a compound that regulates the activity of the protein of [2], comprising steps (a) to (c) below:
   (a) contacting a test compound with said protein;
   (b) determining the activity of said protein; and
   (c) selecting a compound which increases or decreases the activity of said protein in comparison with a case where no test compound is administered.
[21] A method of screening for a compound that regulates the expression level of a gene encoding the protein of [2], comprising steps (a) to (d) below:
   (a) providing a cell or cell extract comprising a DNA in which a reporter gene is functionally bound downstream of a promoter region of a gene encoding said protein;
   (b) contacting a test compound with said cell or cell extract;
   (c) determining the expression level of said reporter gene in said cell or cell extract; and
   (d) selecting a compound which increases or decreases the expression level of said reporter gene in comparison with a case where no test compound is administered.
[22] A method of screening for a compound that regulates the expression level of a gene encoding the protein of [2], comprising steps (a) to (c) below:
   (a) contacting a test compound with an insect individual or tissue culture;
   (b) determining the expression level of the gene encoding the protein of [2] in said insect individual or tissue culture; and
   (c) selecting a compound which increases or decreases the expression level of said gene in comparison with a case where no test compound is administered.

For *Bombyx mori, Drosophila melanogaster, Anopheles gambiae, Spodoptera litura,* and *Helicoverpa armigera,* the present inventors succeeded in isolating DNAs encoding proteins with juvenile hormone acid methyltransferase activities (Juvenile hormone acid methyltransferase: JHAMT). Based on this finding, the present invention provides juvenile hormone acid methyltransferase proteins and DNAs encoding the proteins.

SEQ ID NOs: 1, 3, 5, 7, and 9 show nucleotide sequences of genes encoding juvenile hormone acid methyltransferases of *Bombyx mori, Drosophila melanogaster, Anopheles gambiae, Spodoptera litura,* and *Helicoverpa armigera,* respectively. SEQ ID NOs: 2, 4, 6, 8, and 10 respectively show the anticipated amino acid sequences of the juvenile hormone acid methyltransferases of the above-mentioned insects.

The juvenile hormone acid methyltransferases of the present invention are enzymes that transfer the methyl group of S-adenosylmethionine to the carboxyl group of hormone activity-free juvenile hormone acid, to produce the active juvenile hormone. Thus, the juvenile hormone acid methyltransferases of the present invention can be used to produce natural juvenile hormones. The natural juvenile hormones produced by the juvenile hormone acid methyltransferases of the present invention decompose in ultraviolet light and the like more readily than synthetic juvenile hormones, and thus they are advantageous in that they have low residual toxicity.

A number of methods for chemically synthesizing natural juvenile hormones have been established, but require a large number of steps. On the contrary, the juvenile hormone acid methyltransferases of the present invention are advantageous in that natural juvenile hormones can be produced under physiological conditions. For example, natural type juvenile hormones can be produced using the juvenile hormone acid methyltransferases of the present invention, using the methods described in the Examples. Some kinds of bacteria are known to synthesize farnesoic acid (Oh, K.-B., H. Miyazawa, et al. (2001). Proc Natl Acad Sci USA 98: 4664-4668). Consequently, natural juvenile hormones (methyl farnesoate) can be produced by introducing the enzyme genes of the present invention to such microorganisms. Further, in addition to the above-mentioned bacteria, cultured cells of *Drosophila melanogaster* (Kc cells) or rat liver extracts are known to synthesize farnesoic acid from farnesyl pyrophosphate and farnesol (Gonzalez-Pacanowska, D. et al., 1988: J. Biol. Chem., 1301-1306). Thus, by introducing the enzyme genes of the present invention, natural juvenile hormones may be produced. Further, farnesol is a component in the essential oils of various plants. If a plant further comprises oxidized farnesoic acid, an enzyme gene of the present invention can be introduced to the plant to enable production of natural juvenile hormones.

The natural juvenile hormones produced by the juvenile hormone acid methyltransferases of the present invention can be used in applications well known to persons skilled in the art. Examples of the application of natural juvenile hormones are shown below, but are not limited thereto.

The natural juvenile hormones produced by the juvenile hormone acid methyltransferases of the present invention can be used as pest control agents or cocoon-promoting agents, similarly to juvenile hormone analogous substances. Further, the natural juvenile hormone produced by the juvenile hormone acid methyltransferase of the present invention can be used to regulate insect growth. For example, a natural juvenile hormone can be administered to a mature Pentatomidae in diapause, to terminate the diapause and promote reproductive maturation. Worker honey-bees are known to change their type of work with increasing age. This is thought to be caused by the change in concentration of juvenile hormone with increasing age. Thus, the natural juvenile hormones produced by the juvenile hormone acid methyltransferases of the present invention can be administered from outside, to change the worker-bees' type of work. Further, by treating the larvae of honeycomb moths with a natural juvenile hormone produced by a juvenile hormone acid methyltransferase of the present invention, the larvae can not only be increased in size for successful fishing, but also to inhibit it from pupating, allowing the larvae to be used for a long time (Patent No. 2707617).

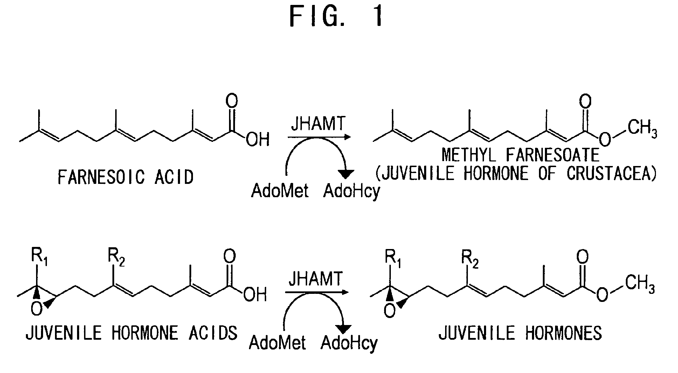

Methyl farnesoate in crustaceans is a hormone homologous to juvenile hormone in insects, and is thought to be important to the growth and reproduction of crustaceans, as it is in insects (Homola, E and Chang, E.S; Comp. Biochem. Physiol. B, 117:347-356, 1997). The juvenile hormone acid methyltransferases of the present invention can also transfer a methyl group to farnesoic acid, which is a juvenile hormone precursor in crustaceans, thus generating methyl farnesoate, an active type hormone. Fig. 1 shows the structures of JH I, JH II, and JH III which are the main juvenile hormones of insects; methyl farnesoate, which is a juvenile hormone of crustaceans; and JH I acid, JH II acid, JH III acid, and farnesoic acid, which are the precursors thereof; as well as a reaction for synthesizing a juvenile hormone from a juvenile hormone acid using an enzyme of the present invention. Therefore, the natural juvenile hormones produced by the juvenile hormone acid methyltransferases of the present invention can also be used to regulate the growth of crustaceans. For example, a juvenile hormone can be used for acorn barnacles, or to improve the growth or collect the eggs of shrimps or crabs cultivated for food. The juvenile hormones can also be used to breed acorn barnacles, one kind of a crustacean (Unexamined Published Japanese Patent Application No. Hei 11-018614).

Juvenile hormones are known to have multiple physiological effects on insect molting/metamorphosis, reproduction, diapause, blastogenesis, behavior, polymorphism, life span, etc. Additionally, juvenile hormones are known to have considerably different physiological actions, even for the same species of insect, depending on growth stage or physiological situation. In particular, the hormones are known to have apparently opposite actions: inhibition of larvae or pupae metamorphosis, and conversely, promotion of reproductive maturation of adult insects. Further, the hormones have different effects on the diapause of an insect, depending on the growing stage in which the diapause occurs. For example, insects which diapause at the larvae stage (such as *Chilo suppressalis),* diapause because the juvenile hormone is retained in the body at high levels, while insects which diapause at the adult stage (such as *Riptortus clavatus)* diapause because of low levels of juvenile hormone. Therefore, when inducing a DNA encoding a juvenile hormone acid methyltransferase of the present invention in insect bodies, the effect on molting/metamorphosis, reproduction, diapause, blastogenesis, behavior, polymorphism, or life span is different, depending on the species, the growth stage, or the physiological situation of the insect, and can be promoting, or inhibiting. Thus, a DNA encoding a juvenile hormone acid methyltransferase of the present invention can be used in a wide range of applications as an agent (promoter or inhibitor) for regulating insect molting/metamorphosis, reproduction, diapause, blastogenesis, behavior, polymorphism, or life span. For example, a DNA encoding a juvenile hormone acid methyltransferase of the present invention can be used as a reproductive maturation promoting agent, a diapause terminating agent, or a life span shortening agent for adult insects; as a metamorphosis inhibiting agent for larvae and pupae (an agent for preserving larvae and pupa characteristics, preferably an extra-molting agent); or a diapause-inducing agent for larvae, but is not limited to these applications.

The DNAs encoding juvenile hormone acid methyltransferases of the present invention may be used as shown below, but uses are not limited thereto.

In a final instar larva where juvenile hormone synthesis ceases, the juvenile hormone acid methyltransferases of the present invention can be induced to continue juvenile hormone synthesis/secretion, enabling extra-molting of the larva. For example, when extra-molting is induced in *Bombyx mori,* it is known to become a large pupa during the 6th instar larva stage, though it usually pupates at the 5th instar larva stage. Therefore, by inducing a juvenile hormone acid methyltransferase of the present invention, a large cocoon can be obtained. A DNA encoding a juvenile hormone acid methyltransferase of the present invention can be used as a cocoon-promoting agent. Further, a juvenile hormone acid methyltransferase of the present invention can be induced in other insects to obtain larger insects, and thus it is useful for producing larger and better looking individuals. For example, it can be applied in the production of pet insects, such as large stage beetles or beautiful butterflies (such as Omithopteras). Furthermore, the juvenile hormone acid methyltransferases of the present invention can be induced in honeybees to improve the production and quality of honey.

A DNA encoding a juvenile hormone acid methyltransferase of the present invention can also be used as pest control agent. For example, a juvenile hormone acid methyltransferase of the present invention can be induced to control medically significant pests such as flies and mosquitoes. Namely, agricultural/forest pests or medically significant pests may be controlled by disturbing the juvenile hormone balance within an insect's body, thus disturbing the physiological functions essential for its survival. Further, a juvenile hormone acid methyltransferase of the present invention can be induced to prevent: egg eclosion (ovicidal action), larvae metamorphosis, pupae emergence and later reproduction, and reproduction of adult insects (in the case of female insects, the number of eggs laid and the rate of eggs hatched will be reduced). In addition, depending on the blastogenesis stage, there is a stage when juvenile hormone is not induced. The juvenile hormone acid methyltransferases of the present invention can be induced in such a stage, to inhibit blastogenesis and egg eclosion (ovicidal action).

Aphididaes and Delphacidaes have polymorphism in their wing type, and both winged and wingless forms exist. In such insects, a juvenile hormone acid methyltransferase of the present invention can be induced to change them into their wingless form.

The DNAs encoding the juvenile hormone acid methyltransferases of the present invention can be used as agents for regulating the molting/metamorphosis, reproduction, diapause, blastogenesis, behavior, polymorphism, or life span of arthropods other than insects. Such arthropods include, but are not limited to, Crustacea, Arachnida, and Myriapoda.

The amount of juvenile hormone (methyl farnesoate) in edible shrimps or crabs can be increased to promote molting and accelerate growth, resulting in efficient cultivation. Similarly, the hormone can promote the reproductive maturation of males and females, and allows the cultivation of species that have been difficult to culture due to difficulty in collecting their eggs.

Plants that synthesize JH III are known (Toong, Y. C., D. A. Schooley, et al. (1988). Isolation of insect juvenile hormone III from a plant. Nature 333: 170-171.). Because grasshoppers that eat such plants undergo abnormal metamorphosis, it is thought that plants with JH have insect resistance. Thus, the genes of the present invention that cause plants to synthesize JH can be used to prepare insect-resistant plants.

Examples of the DNAs of the present invention are the DNAs (SEQ ID NOs: 1, 3, 5, 7, and 9) encoding the juvenile hormone acid methyltransferase of *Bombyx mori, Drosophila melanogaster, Anopheles gambiae, Spodoptera litura,* and *Helicoverpa armigera.*

The DNAs of the present invention comprise DNAs encoding proteins that are functionally equivalent to proteins comprising the amino acid sequences of SEQ ID NOs: 2, 4, 6, 8, and 10. Such DNAs comprise DNAs encoding mutants, alleles, variants, and homologs of the proteins comprising the amino acid sequences of SEQ ID NOs: 2, 4, 6, 8, and 10, or the DNAs encoding the proteins which are mutants, alleles, variants, and homologs of the proteins comprising the amino acid sequences of SEQ ID NOs: 2, 4, 6, 8, and 10, and which have at least one (preferably all) of the motif domains described in Example 7. The term "functionally equivalent" stated herein means that a protein of interest has equivalent biological functions (biological properties) and biochemical functions (biochemical activities) to those of a juvenile hormone acid methyltransferase of *Bombyx mori, Drosophila melanogaster, Anopheles gambiae, Spodoptera litura,* and *Helicoverpa armigera.*

Other well-known methods for preparing a DNA encoding a protein functionally equivalent to another protein comprise methods using hybridization techniques (Sambrook, J et al., Molecular Cloning 2nd ed., 9.47-9.58, Cold Spring Harbor Lab. press, 1989). Namely, for skilled in the art, isolating a DNA sequence highly homologous to a DNA sequence encoding a juvenile hormone acid methyltransferase of *Bombyx mori, Drosophila melanogaster, Anopheles gambiae, Spodoptera litura,* or *Helicoverpa armigera,* by using their DNA sequences, or a part thereof is a well-known technique.

The present invention comprises DNAs which hybridize under stringent conditions with DNAs encoding a juvenile hormone acid methyltransferase of *Bombyx mori, Drosophila melanogaster, Anopheles gambiae, Spodoptera litura,* or *Helicoverpa armigera,* and a DNA encoding a protein functionally equivalent to a juvenile hormone acid methyltransferase of *Bombyx mori, Drosophila melanogaster, Anopheles gambiae, Spodoptera litura,* or *Helicoverpa armigera.* Such DNAs include, but are not limited to, homologs derived from Insecta, Crustacea, Arachnida, Acarina, or Myriopoda (such as Scolopendromorpha, Diplopoda, and *Thereuonema tuberculata).*

While the insects are not limited to any particular insect, beneficial insects include *Bombyx mori* and wild silkworms (such as Saturniidae, *Samia cynthia ricini, Caligura japonica),* and honey bees. Pet insects include Allomyrina and *Dorcus curvidens.* Natural predators include lady bugs and anthocorid bugs. Agricultural pests include *Spodoptera litura, Helicoverpa armigera, Plutella xylotella, Mamestra brassicae,* Aphididae (such as *Aphis gossypii* and *Myzus persicae),* whiteflies (such as *Trialeurodes Vaporariorum* and *Bemisia argentifolli),* Thysanoptera (such as *Thrips palmi* and *Frankliniella occidentalis),* Agromyzidae (such as *Liriomyza trifolli* and *Liriomyza bryoniae),* Pentatomidae (such as *Riptorus clavatus),* Delphacidae (such as *Nilaparuvata lugens, Sogatella furcifera,* and *Laodelphax stratella),* and leafhoppers (such as *Nephotettix cincticeps),* Cerambycidae, *Dacus cucurbitae, Ceratitis capitata.* Forest pests include *Monochamus alternatus* and *Dendrolimus spectabilis.* Domestic livestock pests include Tabanidae (such as *Chrysops suavis* and *Tabanus trigonus),* Diptera (such as *Stomoxys calcitrans* and *Haematobia irritans),* Culcidae, Simuliidae, and Ceratopogonidae. House pests include Isoptera. Medically significant pests include Culcidae (such as *Culex pipiens* and *Aedes albopictus),* Diptera (such as *Drosophila melanogaster, Musca domestica,* Calliphoridae, and Lucilia), Blattiae, Siphonaptera, and Anoplura.

Crustacea in the present invention are not limited to any particular crustacea, and include edible shrimps and crabs (for example, shrimps such as *Peneus japonicus, Homarus americanus,* and *Macrobrachium nipponense;* and crabs such as *Erimacrus isenbeckii, Portunus trituberculatus, Macrocheira kaempferi,* and *Ranina ranina;* francolin; hermit crab; and coconut crab).

Hybridization conditions for isolating a DNA encoding a protein functionally equivalent to a juvenile hormone acid methyltransferase of *Bombyx mori, Drosophila melanogaster, Anopheles gambiae, Spodoptera litura,* or *Helicoverpa armigera* can be appropriately selected by one skilled in the art. The hybridization conditions comprise stringent conditions. Herein, the term "stringent conditions" means conditions that allows formation of specific hybrids without the formation of nonspecific hybrids. One embodiment of stringent conditions in the present invention comprises conditions of low stringency. The conditions of low stringency are, for example, 42°C, 5x SSC and 0.1% SDS for washing after hybridization, preferably 50°C, 5× SSC and 0.1% SDS. More preferable conditions comprise highly stringent conditions. Highly stringent condition are, for example, 65°C, 0.1 × SSC and 0.1 % SDS. Higher temperatures in these conditions are expected to efficiently provide DNAs with higher homology. However, there are multiple elements, such as temperature and salt concentration, which influence the stringency of hybridization. Hence, those skilled in the art can select these elements appropriately to achieve similar stringencies. Another embodiment of the stringent conditions in the present invention comprises, for example, conditions that allow hybridization between nucleic acids with homology as high as 99.5% or more, but that do not allow hybridization between nucleic acids with homology lower than that mentioned above.

Gene amplification methods, such as polymerase chain reactions (PCR), using primers synthesized based on the sequence information of DNAs encoding the juvenile hormone acid methyltransferases of *Bombyx mori, Drosophila melanogaster, Anopheles gambiae, Spodoptera litura,* or *Helicoverpa armigera* can be used to isolate a DNA encoding a protein functionally equivalent to a juvenile hormone acid methyltransferase *of Bombyx mori, Drosophila melanogaster, Anopheles gambiae, Spodoptera litura,* or *Helicoverpa armigera.*

A protein functionally equivalent to a juvenile hormone acid methyltransferase of *Bombyx mori, Drosophila melanogaster, Anopheles gambiae, Spodoptera litura,* or *Helicoverpa armigera,* encoded by a DNA isolated according to a hybridization technique and gene amplification technique, generally has high amino acid sequence homology to a juvenile hormone acid methyltransferase of *Bombyx mori, Drosophila melanogaster, Anopheles gambiae, Spodoptera litura,* or *Helicoverpa armigera.* Proteins of the present invention comprise proteins functionally equivalent to the juvenile hormone acid methyltransferase of *Bombyx mori, Drosophila melanogaster, Anopheles gambiae, Spodoptera litura,* or *Helicoverpa armigera,* and proteins with high amino acid sequence homology to that protein. High homology generally refers to at least 50% identity, preferably 75% or more identity, more preferably 85% or more identity, even more preferably 95% or more identity, and most preferably 98% or more identity on the amino acid level; or generally refers to at least 50% identity, preferably 75% or more identity, more preferably 85% or more identity, even more preferably 95% or more identity, and most preferably 98% or more identity on the nucleotide level.

The degree of homology of one amino acid sequence or nucleotide sequence to another can be determined by following the BLAST algorithm by Karlin and Altschl (Proc. Natl. Acad. Sci. USA, 90: 5873-5877, 1993). Programs such as BLASTN and BLASTX were developed based on this algorithm (Altschul et al. J. Mol. Biol.215: 403-410, 1990). To analyze a nucleotide sequence according to BLASTN, based on BLAST, the parameters are set as score= 100 and word length= 12, for example. On the other hand, parameters used for the analysis of amino acid sequences by BLASTX based on BLAST include, for example, score= 50 and word length= 3. The default parameters of each program are used when using BLAST and Gapped BLAST programs. Specific techniques for such analysis are known in the art (http://www.ncbi.nlm.nih.gov.)

The present invention comprises proteins that comprise the amino acid sequence of a juvenile hormone acid methyltransferase of *Bombyx mori, Drosophila melanogaster, Anopheles gambiae, Spodoptera litura,* or *Helicoverpa armigera* with one or more amino acids mutated, and that are functionally equivalent to that protein. Such amino acid mutations occur in nature. The number of mutated amino acids varies depending on the sites of addition, deletion, or substitution of amino acid residues within a juvenile hormone acid methyltransferase protein, or the oils of amino acid residue. The number of mutated amino acids is preferably 30 or less, more preferably 2 to 20, and more preferably about 2 to 15 amino acids.

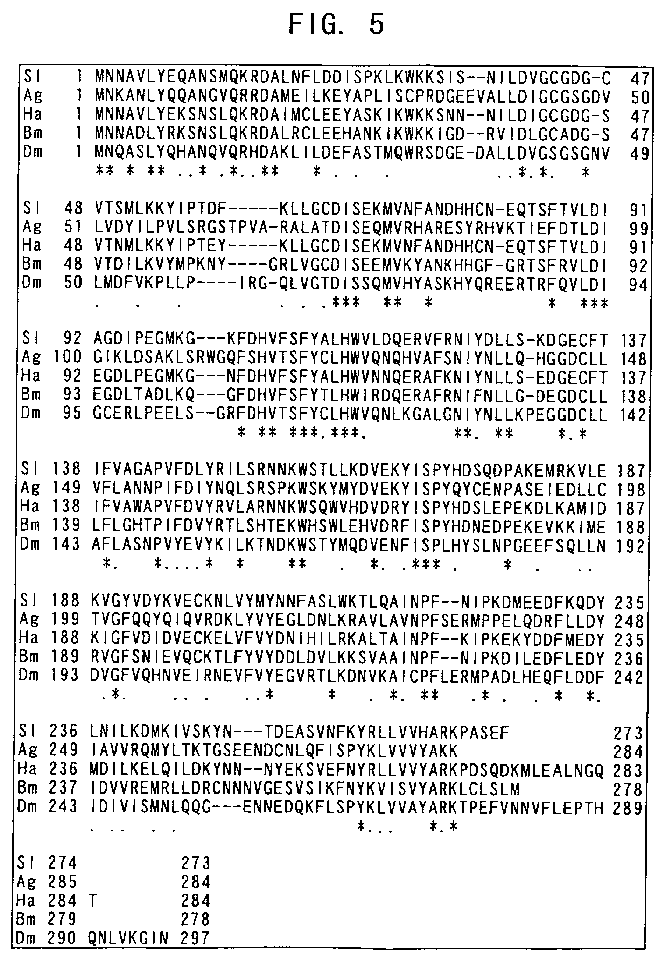

The sites of amino acid mutation are not particularly limited, but are preferably sites other than the characteristic motif sites described in the Examples, and other than the highly preserved amino acid sites. Sites other than the characteristic motif sites and highly preserved amino acid sites can be determined by reference to Example 7 and Fig. 5d.

An amino acid residue is preferably mutated into an amino acid residue that conserves the properties of the amino acid side chain. Examples of amino acid side chain properties are: hydrophobic amino acids (A, I, L, M, F, P, W, Y, V), hydrophilic amino acids (R, D, N, C, E, Q, G, H, K, S, T), and amino acids comprising the following side chains: aliphatic side chains (G, A, V, L, I, P); hydroxyl group-containing side chains (S, T, Y); sulfur atom-containing side chains (C, M); carboxylic acid- and amide-containing side chains (D, N, E, Q); base-containing side chains (R, K, H); and aromatic-containing side chains (H, F, Y, W) (the letters within parenthesis are the one-letter amino acid codes).

It is well known that a protein comprising a modified amino acid sequence in which one or more amino acid residues is deleted, added, and/or substituted can retain its original biological activity (Mark D.F. et al., Proc. Natl. Acad. Sci. U.S.A. 81: 5662-5666 (1984); Zoller M.J. and Smith M., Nucleic Acids Res. 10: 6487-6500 (1982); Wang A. et al., Science 224: 1431-1433; Dalbadie-McFarland G. et al., Proc. Natl. Acad. Sci. U.S.A. 79: 6409-6413 (1982)).

In addition, one method well known to those skilled in the art for preparing proteins functionally equivalent to a specific protein is to introduce mutations into the protein. For example, one skilled in the art can prepare polypeptides functionally equivalent to a protein by introducing appropriate mutations into the amino acid sequence of juvenile hormone acid methyltransferase of *Bombyx mori, Drosophila melanogaster, Anopheles gambiae, Spodoptera litura,* or *Helicoverpa armigera,* through site-specific mutagenesis or the like (Hashimoto-Gotoh, T. et al. (1995) Gene 152, 271-275; Zoller, MJ, and Smith, M. (1983) Methods Enzymol. 100, 468-500; Kramer, W. et al. (1984) Nucleic Acids Res. 12, 9441-9456; Kramer W, and Fritz HJ (1987) Methods. Enzymol. 154, 350-367; Kunkel, TA (1985) Proc Natl Acad Sci U S A. 82, 488-492; Kunkel (1988) Methods Enzymol. 85,2763-2766).

A modified DNA can also be obtained by carrying out mutation treatment on a cell that comprises a DNA of the present invention, and then from that cell, selecting DNAs that hybridize under stringent conditions with a DNA encoding the juvenile hormone acid methyltransferase of *Bombyx mori, Drosophila melanogaster, Anopheles gambiae, Spodoptera litura,* or *Helicoverpa armigera,* for example.

Proteins comprising multiple amino acid residues added to the amino acid sequence of a juvenile hormone acid methyltransferase of *Bombyx mori, Drosophila melanogaster, Anopheles gambiae, Spodoptera litura,* or *Helicoverpa armigera* include fusion proteins comprising these proteins. Fusion proteins are fusions of these proteins and other proteins, and are comprised in the present invention. To prepare a fusion protein, for example, a DNA encoding a juvenile hormone acid methyltransferase of *Bombyx mori, Drosophila melanogaster, Anopheles gambiae, Spodoptera litura,* or *Helicoverpa armigera* may be linked to a DNA encoding another protein such that their frames correspond, then inserted into an expression vector, and expressed in a host. The proteins to be fused with the proteins of the present invention are not particularly limited.

Known peptides are, for example, FLAG (Hopp, T.P. et al., Biotechnology (1988) 6, 1204-1210), 6x His containing six His (histidine) residues, 10x His, Influenza agglutinin (HA), human c-myc fragment, VSP-GP fragment, p18HIV fragment, T7-tag, HSV-tag, E-tag, SV40T antigen fragment, 1ck tag, α-tubulin fragment, B-tag, and Protein C fragment, and can be used as peptides that are fused to a protein of the present invention. Examples of proteins that are fused to a protein of the present invention are GST (glutathione-S-transferase), Influenza agglutinin (HA), immunoglobulin constant region, β-galactosidase, and MBP (maltose-binding protein). Fusion proteins can be prepared by fusing a commercially available DNA encoding these proteins with a DNA encoding a protein of the present invention; and then expressing the fused DNA thus prepared.

The DNAs of the present invention may be in any form as long as they can encode a protein of the present invention. Namely, the DNAs may be cDNAs synthesized from mRNAs, genomic DNAs, or chemically synthesized DNAs. Further, DNAs with an arbitrary nucleotide sequence based on the degeneracy of the genetic code are also comprised in the present invention, so long as they can encode a protein of the present invention.

The DNAs of the present invention can be prepared by methods known to those skilled in the art. For example, a cDNA library is produced from the corpora allata, gonad, imaginal disc, entire insect, or entire insect head, the mandibular organ of a Crustacea (an organ for synthesizing methyl farnesoate), the entire body or entire head of a Crustacea, or the entire body or entire head of an Arachnida, Acaria, or Myriapoda. Then, part of a DNA encoding a juvenile hormone acid methyltransferase of *Bombyx mori, Drosophila melanogaster, Anopheles gambiae, Spodoptera litura,* or *Helicoverpa armigera* is used as a probe for hybridization, enabling the preparation of naturally derived DNA. Alternatively, RNAs are prepared from the corpora allata, gonad, imaginal disc, entire insect, or entire insect head, the mandibular organ of a Crustacea (an organ for synthesizing methyl farnesoate), the entire body or entire head of a Crustacea, or the entire body or entire head of an Arachnida, Acaria, or Myriapoda. A reverse transcriptase is then used to synthesize cDNAs. Oligo DNAs are synthesized based on the DNAs encoding the juvenile hormone acid methyltransferase of *Bombyx mori, Drosophila melanogaster, Anopheles gambiae, Spodoptera litura,* or *Helicoverpa armigera,* and then used as primers to carry out PCR reactions. Thus, the DNAs of the present invention can be prepared by amplifying cDNAs encoding the proteins of the present invention.

For instance, total RNA can be prepared by known methods, for example guanidine ultracentrifugation (Chirgwin J.M. et al. Biochemistry 18:5294-5299 (1979)) or AGPC methods (Chomczynski P. and Sacchi N. Anal. Biochem. 162:156-159 (1987)), and mRNA can be purified from total RNA using an mRNA Purification Kit (Pharmacia) or such. Alternatively, mRNA may be directly prepared using a QuickPrep mRNA Purification Kit (Pharmacia).

The obtained mRNAs are used to synthesize cDNAs using reverse transcriptase. cDNAs may be synthesized using a kit such as the AMV Reverse Transcriptase First-strand cDNA Synthesis Kit (Seikagaku Kogyo). Alternatively, cDNAs may be synthesized and amplified following the 5'-RACE method (Frohman M.A. et al., Proc. Natl. Acad. Sci. U.S.A. 85: 8998-9002 (1988); Belyavsky A. et al., Nucleic Acids Res. 17: 2919-2932 (1989)), using the 5'-Ampli FINDER RACE Kit (Clontech) and polymerase chain reaction (PCR).

The present invention provides proteins encoded by the above-mentioned DNAs of the present invention. The proteins of the present invention may vary in their amino acid sequence, molecular weight, isoelectric point, or the presence or configuration of saccharide chains, depending on the cell or host for production, or method for purification. However, the proteins thus obtained are comprised in the present invention, as long as they are functionally equivalent to a juvenile hormone acid methyltransferase of *Bombyx mori, Drosophila melanogaster, Anopheles gambiae, Spodoptera litura,* or *Helicoverpa armigera.* For example, when a protein of the present invention is expressed in a prokaryotic cell (e.g. *Escherichia coli),* a methionine residue is added at the N terminus of the amino acid sequence of the original protein. The proteins of the present invention also comprise such proteins.

Using methods known to those skilled in the art, the proteins of the present invention can be prepared as recombinant or natural proteins. If recombinant proteins, a system for expressing and recovering large amounts of a protein of the present invention may be used, using a host cell (such as *Escherichia coli* or yeast) introduced with a DNA encoding a protein of the present invention, or cultured cells or insect bodies infected with a recombinant Baculovirus transfected with the DNA. A protein of the present invention can be prepared and purified using chromatographies such as ion-exchange chromatography, reverse phase chromatography, and gel filtration chromatography, or affinity chromatography using an antibody against the protein of the present invention immobilized on the column, or by further combining multiple columns.

When a protein of the present invention is expressed in host cells (such as animal cells or *Escherichia coli)* as a fusion protein with glutathione S-transferase protein, or as a recombinant protein with multiple histidines added, the recombinant protein thus expressed can be purified using a glutathione column or nickel column. After purifying the fusion protein, regions other than the protein of interest can be removed from the fusion protein by cleavage with thrombin or factor-Xa, as necessary.

Natural proteins can be isolated by purification methods well known to those skilled in the art, for example, by applying an extract from the corpora allata of an insect to an affinity column bound with an antibody against a protein of the present invention (discussed later). The antibody may be a polyclonal antibody or a monoclonal antibody.

The present invention provides DNAs that inhibit the expression of the above-mentioned DNAs encoding juvenile hormone acid methyltransferase. Such DNAs comprise DNAs encoding antisense RNAs of a DNA encoding juvenile hormone acid methyltransferase, DNAs encoding RNAs with ribozyme activity that specifically cleaves a transcription product of a DNA encoding juvenile hormone acid methyltransferase, and DNAs encoding RNAs which inhibit the expression of a DNA encoding juvenile hormone acid methyltransferase using an RNAi effect.

The DNAs that inhibit the expression of a DNA encoding juvenile hormone acid methyltransferase can also be used as agents for regulating the molting/metamorphosis, reproduction, diapause, blastogenesis, behavior, polymorphism, or life span of arthropods (preferably insects), similarly to the DNAs encoding the juvenile hormone acid methyltransferase. Namely, when a DNA that inhibits expression of a DNA encoding juvenile hormone acid methyltransferase is induced in an insect body, its influences on the molting/metamorphosis, reproduction, diapause, blastogenesis, behavior, polymorphism, or life span will vary depending on the type of insect, its growing stage, or its physiological situation, and can be accelerative or suppressive. For example, and without limitation, DNAs that inhibit expression of a DNA encoding juvenile hormone acid methyltransferase can be used as reproductive maturation inhibiting agents, diapause inducing agents, or life span extending agents for adult insects; or diapause inhibiting agents or metamorphosis inducing agents (preferably early metamorphosis inducing agents) for larvae.

Applications of the DNAs of the present invention that inhibit the expression of DNAs encoding juvenile hormone acid methyltransferases are described below, but are not limited to the following examples.

Metamorphosis is known to start because the activity of juvenile hormone acid methyltransferase decreases in final instar larva, and thus synthesis and secretion of juvenile hormone ceases, and it disappears from the blood. Therefore, early metamorphosis can be induced at will in insects (both holometabolous or hemimetabolous insects) in a larvae stage before the final instar by using the above-mentioned DNAs to inhibit synthesis of juvenile hormone. For example, when the corpora allata, an organ for secreting juvenile hormone, is removed in *Bombyx mori* to induce its early metamorphosis, it pupates at the 3rd or 4th instar stage, instead of ordinary pupation at the 5th instar stage, and an extremely small adult insect may be obtained. Adult insects that have undergone early metamorphosis are generally sterile. Accordingly, insects in succeeding generations can be exterminated by inhibiting juvenile hormone synthesis using the above-mentioned DNAs. For example, the larvae of agricultural pests or medically significant pests can be induced to undergo early metamorphosis, shortening the term during which they do harm, and enabling control of the pests. Further, the reproductive maturation (such as oogenesis) of adult insects may be inhibited to enable control of agricultural or medically significant pests. Thus, the DNAs of the present invention, which inhibit the expression of a DNA encoding a juvenile hormone acid methyltransferase, can be used as pest control agents.

Insect pests in the present invention are as follows: Agricultural pests include *Spodoptera litura, Helicoverpa armigera, Plutelae xylotella, Mamestra brassicae,* aphids (such as *Aphis gossypii* and *Myzus persicae),* whiteflies (such as *Trialeurodes Vaporariorum* and *Bemisia argentifolli),* Thysanoptera (such as *Thrips palmi* and *Frankliniella occidentalis),* Agromyzidae (such as *Liriomyza trifolli* and *Liriomyza bryoniae),* Pentatomidae (such as *Riptorus clavatus),* Delphacidae (such as *Nilaparuvata lugens, Sogatella furcifera,* and *Laodelphax stratella),* leafhoppers (such as *Nephotettix cincticeps),* Cerambycidae, *Dacus cucurbitae, Ceratitis capitata.* Forest pests include *Monochamus alternatus* and *Dendrolimus spectabilis.* Domestic livestock pests include Tabanidae (such as *Chrysops suavis* and *Tabanus trigonus),* Diptera (such as *Stomoxys calcitrans* and *Haematobia irritans),* Culcidae, Simuliidae, and Ceratopogonidae. House pests include Isoptera. Medically significant pests include Culcidae (such as *Culex pipiens* and *Aedes albopictus),* Diptera (such as *Drosophila melanogaster, Musca domestica,* Calliphoridae, and Lucilia), Blattiae, Siphonaptera, and Anoplura. However, pest insects are not limited to these.

By using the above-mentioned DNAs to inhibit juvenile hormone synthesis, inhibition of reproductive maturation and induction of diapause can be induced in adult insects such as Pentatomidae. Aphididaes and Delphacidaes have polymorphism in their wing type (winged and wingless forms). The above-mentioned DNAs can be expressed in Aphididaes or Delphacidaes larvae that naturally change into wingless forms, to produce their winged forms. In insects such as *Drosophila melanogaster* or *Danaus plexippus,* a phenomenon is known wherein ceasing juvenile hormone secretion at the adult stage causes the insect to fall into a diapause-like situation, allowing reproductive maturation to stop and life span to extend. Therefore, in these insects, inhibition of juvenile hormone synthesis using an above-mentioned DNA can extend life span. By applying this phenomenon, the above-mentioned DNAs can be used to inhibit juvenile hormone synthesis, and natural predator insects (for example ladybugs) and the like may be put into diapause at will, to allow long-term storage. The concentration of juvenile hormone in worker honeybees is known to increase as they age, changing their work content (behavior) (Hiromi Sasagawa (1999) Kankyou Konchugaku - Koudou, Seiri, Kagaku Seitai (Environmental Entomology-Behavior/Physiology/Chemical Ecology), University of Tokyo Press, p143-172). Therefore, by inhibiting juvenile hormone synthesis, the behavior of worker honeybees can be regulated.

There are a number of factors, indicated below, in the action of antisense nucleic acids in inhibiting the expression of a target gene. Examples comprise: inhibition of transcription initiation by forming a triple strand, inhibition of transcription by hybridizing with a site of an open loop structure locally formed by an RNA polymerase, inhibition of transcription by hybridizing with an RNA with ongoing synthesis, inhibition of splicing by hybridizing at the conjugating point of an intron and an exon, inhibition of splicing by hybridizing with a site for spliceosome formation, inhibition of transition from nucleus to cytoplasm by hybridizing with an mRNA, inhibition of splicing by hybridizing with a capping site or poly(A) addition site, inhibition of translation initiation by hybridizing with a binding site of a translation initiation factor, inhibition of translation by hybridizing with a binding site of a liposome around an initiation codon, inhibition of the extension of a peptide chain by hybridizing with a translation domain of an mRNA or a binding site of a polysome, and inhibition of gene expression by hybridizing with an interaction site of a nucleic acid and a protein. Thus, an antisense nucleic acid inhibits various processes such as transcription, splicing, or translation, as mentioned above, thereby inhibiting expression of a target gene (Hirajima and Inoue: Shin Seikagaku Jikken Kouza 2 (Biochemistry Experiment Course), Nucleic acid IV, Replication and Expression of Genes (edited by The Japanese Biochemical Society, Tokyo Kagaku Dozin Co., Ltd.) pp.319-347, 1993).

The antisense sequences used in the present invention may inhibit the expression of a target gene by any of the actions mentioned above. In one embodiment, to allow efficient inhibition of gene translation, an antisense sequence may be designed to be complementary to a noncoding region near the 5' end of an mRNA for a gene. Alternatively, sequences complementary to a coding or noncoding region of the 3' side can also be used. As such, the antisense DNAs used in the present invention comprise DNAs that comprise antisense sequences to sequences not only for coding regions but also for noncoding regions of a gene. The antisense DNAs used are linked downstream of a suitable promoter, and a sequence comprising a transcription termination signal is preferably linked to the 3' side. DNAs thus prepared can be used to transform a desirable organism by known methods. The antisense DNA sequences are preferably sequences complementary to an endogenous gene, or part thereof, of an organism to be transformed, however, as long as they can efficiently inhibit gene expression, they do not need to be completely complementary. A transcribed RNA is preferably 90% or more complementary, and most preferably 95% or more complementary to the transcript of a target gene. To use an antisense sequence to efficiently inhibit the expression of a target gene, the antisense DNA has a length of at least 15 nucleotides or more, preferably 100 nucleotides or more, and more preferably 500 nucleotides or more. Antisense DNAs in general used are shorter than 5kb, and preferably shorter than 2.5kb.

Alternatively, a DNA encoding a ribozyme can be used to inhibit expression of an endogenous gene. A ribozyme refers to an RNA molecule with catalytic activity. Ribozymes with various activities exist. Studies focusing on ribozymes as enzymes for cleaving RNA have enabled the design of ribozymes that site-specifically cleave RNA. Some ribozymes are 400 or more nucleotides long, such as those of the group I intron type, or M1 RNA comprised in RNase P. Others, such as hammerhead-type or hairpin-type ribozymes, have an active domain of about 40 nucleotides(Makoto Koizumi and Eiko Otsuka: Tanpakushitsu Kakusan Kouso (Protein/Nucleic Acid/Enzyme), 35: 2191, 1990).

For example, the self-cleaving domain of the hammerhead-type ribozyme cleaves the 3' side of C15 in the sequence G13U14C15. Base pair formation between U14 and A9 is essential to this activity, but cleavage atA15 or U15 instead of C15 is also possible (Koizumi M, et al: FEBS Lett 228: 228, 1988). If a ribozyme is designed to be complementary to an RNA sequence with a substrate binding site near the target site, an RNA-cleaving ribozyme can be produced that is like a restriction enzyme, that recognizes a UC, UU, or UA sequence within the target RNA (Koizumi M, et al: FEBS Lett 239: 285, 1988; Makoto Koizumi and Eiko Otsuka: Tanpakushitsu Kakusan Kouso (Protein/Nucleic Acid/Enzyme), 35: 2191, 1990; Koizumi M, et al: Nucl Acids Res 17: 7059, 1989). For example, DNAs encoding juvenile hormone acid methyltransferases (SEQ ID NOs: 1, 3, 5, 7, or 9) comprise multiple targeting sites.

Hairpin-type ribozymes are also useful to the objectives of the present invention. These ribozymes are found, for example, in the minus strain of satellite RNA of Tobacco ring spot virus (Buzayan JM: Nature 323: 349, 1986). Hairpin-type ribozymes have been shown to also enable creation of target-specific RNA-cleaving ribozymes (Kikuchi Y & Sasaki N: Nucl Acids Res 19: 6751, 1991; Yo Kikuchi: Kagaku to Seibutsu (Chemistry and Biology) 30: 112, 1992).

To enable transcription in an insect cell at an arbitrary time, ribozymes designed to be able to cleave targets are linked to promoters such as heat shock promoter or tetracycline-responsive promoter, and to transcription termination sequences, for example. At this time, if a surplus sequence is added to the 5' end or the 3' end of the transcribed RNA, the ribozyme will lose its activity. In such cases, in order to precisely cleave out only the ribozyme portion from the RNA comprising the transcribed ribozyme, another cis-acting trimming ribozyme may be located 5' or 3' of the ribozyme portion (Taira K, et al: Protein Eng 3: 733, 1990; Dzianott AM & Bujarski JJ: Proc Natl Acad Sci USA 86: 4823, 1989; Grosshans CA & Cech TR: Nucl Acids Res 19: 3875, 1991; Taira K, et al: Nucl Acids Res 19: 5125, 1991). Further, to increase the effect, such constituting units can be arrayed in tandem to allow cleavage of multiple sites in a target gene (Yuyama N, et al: Biochem Biophys Res Commun 186: 1271, 1992). As mentioned above, ribozymes can be used to specifically cleave the transcript of a target gene of the present invention, thereby inhibiting expression of that gene.

Furthermore, the expression of endogenous genes can also be inhibited by RNA interference (RNAi) by using double-stranded RNAs with the same or a similar sequence to that of a target gene. RNAi indicates a phenomenon whereby a double-stranded RNA having the same or similar sequence to that of a target gene is introduced to a cell to inhibit expression of both the introduced foreign gene and the target endogenous gene. The detailed mechanism of RNAi remains unclear, but it is thought that the initially introduced double-stranded DNA decomposes into small pieces, which somehow change into target indicators and decompose the target gene. RNAi is also known to affect insects (Kennerdell, J.R. & Carthew, R.W.: Nat. Biotechnol. 18:896, 2000; Piccin, A. et al.: Nucleic Acid Res. 29:E55, 2001; Bettencourt, R. et al.: Insect Mol. Biol. 11:267, 2002; Hughes, C.L. & Kaufman, T.C.: Development 127:8683, 2000).

The DNAs of the present invention are preferably used as constructs (Smith, N.A. et al. Nature, 407:319, 2000; Wesley, S.V. et al. Plant J. 27:581, 2001; Piccin, A. et al. Nucleic Acids Res. 29:E55, 2001) for inserting an appropriate sequence (an intron sequence is desirable) between the inverted repeats of a target sequence to form a double-stranded RNA with a hairpin structure (self complementary 'hairpin' RNA (hpRNA)).

A DNA used for RNAi need not be completely identical to a target gene, but has sequence identity of at least 70% or more, preferably 80% or more, more preferably 90% or more, and most preferably 95% or more. The sequence identity can be determined by an above-mentioned technique.

The present invention provides vectors inserted with a DNA of the present invention. Such vectors comprise vectors wherein a DNA of the present invention is bound (functionally bound) downstream of a promoter that can be induced at an arbitrary timing. The term "functionally bound" herein means that the promoter region, which can be induced at an arbitrary time, and the DNA of the present invention are bound such that a transcription factor may bind the promoter domain to induce expression of the DNA of the present invention. Accordingly, even a DNA of the present invention bound to other genes to form a fusion protein with another gene product is included in the meaning "functionally bound", as mentioned above, as long as the transcription factor binds the promoter domain which can be induced at an arbitrary time to induce expression of the fusion protein. Vectors inserted with a DNA of the present invention can be used as an agent for regulating the molting/metamorphosis, reproduction, diapause, blastogenesis, behavior, polymorphism, or life span of arthropods (preferably insects).

Promoters which can be induced at an arbitrary time comprise heat shock promoters or tetracycline-responsive promoters. Vector constructs wherein a DNA of the present invention is bound downstream of a promoter which can be induced at an arbitrary time, such as a heat shock promoter or tetracycline-responsive promoter, can be produced and used with reference to the literature (Uhlirova M et al.: Dev. Genes. Evol., 212:145-151, Bello, B. et al.: Development 125, 2193-2202, 1998).

The vectors of the present invention may be used to retain the DNAs of the present invention in host cells. When *E. coli* is used as a host cell, there is no limitation other than that the vector should have an "ori" to amplify and mass-produce the vector in *E*. *coli* (e.g., JM109, DH5α, HB101, or XL1Blue, and such), and a marker gene for selecting the transformed *E*. *coli* (e.g., a drug-resistance gene selected by a drug, such as ampicillin, tetracycline, kanamycin, or chloramphenicol).

For example, M13-series vectors, pUC-series vectors, pBR322, pBluescript, pCR-Script, and such can be used. Besides the vectors, pGEM-T, pDIRECT, pT7, and such can be also used for subcloning and excision of the cDNAs.

When a vector is used to produce the polypeptides of the present invention, an expression vector is especially useful. When the expression vector is expressed, for example, in *E. coli,* it should comprise the above characteristics in order to be amplified in *E. coli.* Additionally, when *E. coli,* such as JM109, DH5α, HB101, or XL1-Blue, are used as host cells, the vector should have a promoter that allows efficient expression of the desired gene in *E. coli,* e.g. lacZ promoter (Ward et al. (1989) Nature 341:544-546; (1992) FASEB J. 6:2422-2427), araB promoter (Better et al. (1988) Science 240:1041-1043), or T7 promoter. Other examples of the vectors include pGEX-SX-1 (Pharmacia), "QIAexpress system" (QIAGEN), pEGFP, and pET (for this vector, BL21, a strain expressing T7 RNA polymerase, is preferably used as the host).

Furthermore, the vectors may comprise a signal sequence for protein secretion. When producing proteins in to the periplasm of *E*. *coli,* the pelB signal sequence (Lei S.P. et al. J. Bacteriol. 169:4379 (1987)) may be used as a signal sequence for protein secretion. Calcium chloride methods or electroporation may be used to introduce the vectors into host cells.

In addition to using *E. coli,* the proteins of the present invention may also be produced using expression vectors derived from mammals (e.g., pCDNA3 (Invitrogen), pEGF-BOS (Nucleic Acids Res. (1990) 18(17): 5322), pEF, pCDM8); insect cells (e.g., "Bac-to-BAC baculovirus expression system" (GIBCO-BRL), pBacPAK8); plants (e.g. pMH1, pMH2); animal viruses (e.g., pHSV, pMV, pAdexLcw); retroviruses (e.g. pZIPneo); yeasts (e.g., "Pichia Expression Kit" (Invitrogen), pNV11, SP-Q01); and *Bacillus subtilis* (e.g. pPL608, pKTH50).

In order to express proteins in animal cells, such as CHO, COS, and NIH3T3 cells, the vectors must comprise a promoter necessary for expression in such cells (e.g., SV40 promoter (Mulligan et al. (1979) Nature 277: 108), MMLV-LTR promoter, EF1α promoter (Mizushima et al. (1990) Nucleic Acids Res. 18: 5322) and CMV promoter). It is preferable that the vector also comprises a marker gene for selecting transformants (for example, a drug-resistance gene selected by a drug such as neomycin and G418). Examples of vectors with such characteristics include pMAM, pDR2, pBK-RSV, pBK-CMV, pOPRSV, pOP13, and such.

Furthermore, when aiming to stably express a gene and to amplify its copy number in cells, methods can be used that, for example, introduce CHO cells defective in a nucleic acid synthetic pathway with a vector (such as pCHOI) carrying a DHFR gene that compensates for the defect, and then amplify the vector with methotrexate (MTX). Alternatively, when aiming for transient gene expression, examples of methods include those in which COS cells that comprise a gene that expresses the SV40 T antigen in the chromosome are transformed with a vector (such as pcD) carrying an SV40 replication origin. The replication origin may be that of a polyomavirus, adenovirus, bovine papilloma virus (BPV), or the like. Also, to amplify the gene copy number in the host cells, selection markers, such as the aminoglycoside transferase (APH) gene, thymidine kinase (TK) gene, *E*. *coli* xanthine-guanine phosphoribosyl transferase (Ecogpt) gene, and the dihydrofolate reductase (dhfr) gene, may be comprised in the expression vector.

DNAs of the present invention can be expressed in animals by, for example, inserting a DNA of the invention into an appropriate vector and introducing this vector into a living cell via retroviral methods, liposome methods, cationic liposome methods, adenovirus methods, and such. The vectors used in these methods comprise, but are not limited to, adenovirus vectors (e.g., pAdexlcw), retrovirus vectors (e.g. pZIPneo), and such. General gene manipulation techniques, such as inserting a DNA of the present invention into a vector, can be performed according to conventional methods (Molecular Cloning, 5.61-5.63). Administration to a living body may be performed by *ex vivo* or *in vivo* methods.

The present invention also provides host cells into which a vector of the present invention has been introduced. The host cells into which a vector of the present invention is introduced are not particularly limited. For example, *E. coli* and various animal cells can be used. A host cell of the present invention can be used, for example, as a production system to produce and express a protein of the present invention. The systems for producing the proteins comprise *in vitro* and *in vivo* systems. Production systems that use eukaryotic cells or prokaryotic cells are examples of *in vitro* production systems.

Eukaryotic host cells that can be used are, for example, animal cells, plant cells, and fungi cells. Mammalian cells, for example, CHO (J. Exp. Med. (1995) 108:945), COS, 3T3, myeloma, BHK (baby hamster kidney), HeLa, Vero, amphibian cells (e.g., Xenopus oocytes (Valle et al. (1981) Nature 291:358-340), and insect cells (e.g. Sf9, Sf21, Tn5) are known as animal cells. Among CHO cells, those defective in the DHFR gene, dhfr-CHO (Proc. Natl. Acad. Sci. USA (1980) 77:4216-4220) and CHO K-1 (Proc. Natl. Acad. Sci. USA (1968) 60:1275) are particularly preferable. Among animal cells, CHO cells are particularly preferable for large-scale expression. A vector can be introduced into a host cell by, for example, calcium phosphate methods, DEAE-dextran methods, methods using cationic liposome DOTAP (Boehringer-Mannheim), electroporation methods, and lipofection methods.

Plant cells originating from *Nicotiana tabacum* are known as protein-producing systems and may be used as callus cultures. As fungal cells, yeast cells such as *Saccharomyces,* including *Saccharomyces cerevisiae,* or filamentous fungi such as *Aspergillus,* including *Aspergillus niger,* are known and included in the scope of this invention.

Useful prokaryotic cells comprise bacterial cells. Examples of bacterial cells include *E*. *coli,* for example, JM109, DH5α, HB101 and such, and *Bacillus subtilis* are also known.

These cells are transformed by a desired DNA, and the transformants are cultured *in vitro* to obtain a protein. Transformants can be cultured using known methods. For example, the culture medium for animal cells may be a culture medium such as DMEM, MEM, RPMI1640, or IMDM, and may be used with or without serum supplements such as fetal calf serum (FCS). The pH of the culture medium is preferably between about 6 and 8. Such cells are typically cultured at about 30 to 40°C for about 15 to 200 hours, and the culture medium may be replaced, aerated, or stirred as necessary.

Production systems using animal and plant hosts may be used as systems for producing proteins *in vivo.* For example, target DNAs are introduced into these animal or plant hosts, and proteins are produced in the body of the animal or plant, and then recovered. These animals and plants are included in the "hosts" of the present invention.

The animals to be used for the production system described above comprise mammals and insects. Mammals such as goats, pigs, sheep, mice, and cattle may be used (Vicki Glaser, SPECTRUM Biotechnology Applications (1993)). Alternatively, the mammals may be transgenic animals.

For instance, a target DNA may be prepared as a fusion gene with a gene that encodes a protein specifically produced in milk, such as the goat β casein gene. DNA fragments comprising the fusion gene are injected into goat embryos, which are then introduced back to female goats. Target proteins are recovered from the milk produced by the transgenic goats (i.e., those goats born from the goats that received the modified embryos) or from their offspring. Appropriate hormones may be administered to increase the volume of milk comprising the proteins produced by transgenic goats (Ebert K.M. et al. (1994) Bio/Technology 12: 699-702).

Alternatively, insects, such as silkworms, may be used as hosts. Baculoviruses, into which a DNA encoding a target protein has been inserted, can be used to infect silkworms, and the desired protein can be recovered from their body fluids (Susumu M. et al. (1985) Nature 315: 592-594).

In addition, when using plants, tobacco, for example, can be used. When using tobacco, a DNA encoding a desired protein may be inserted into a plant expression vector, such as pMON 530, which is introduced into bacteria, such as *Agrobacterium tumefaciens.* Then, the bacteria are used to infect tobacco, such as *Nicotiana tabacum,* and the desired protein is recovered from the leaves (Julian K. -C. Ma et al. (1994) Eur. J. Immunol. 24: 131-138).

A protein of the present invention, obtained as above, may be isolated from the inside or outside (the medium and such) of host cells, and purified as a substantially pure homogeneous protein. Methods for isolating and purifying proteins are not limited to any specific method; in fact, any standard method for isolating and purifying proteins may be used. For instance, column chromatography, filters, ultrafiltration, salting out, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the proteins.

Chromatography such as affinity chromatography, ion-exchange chromatography, hydrophobic chromatography, gel filtration chromatography, reverse phase chromatography and adsorption chromatography may be used (Strategies for Protein Purification and Characterization: A Laboratory Course Manual. Ed. Daniel R. Marshak et al., Cold Spring Harbor Laboratory Press (1996)). These chromatographies may be performed using liquid chromatographies, such as HPLC and FPLC. Thus, the present invention provides highly purified proteins produced by the above methods.

A protein may also be arbitrarily modified, or peptides may be partially removed, by the action of an appropriate protein modification enzyme on the protein before or after protein purification. Protein modification enzymes such as trypsin, chymotrypsin, lysylendopeptidase, protein kinase, and glucosidase are used.

The present invention provides individuals transformed with a DNA of the present invention or a vector of the present invention. Examples of the individuals comprise the above-mentioned individuals, but are preferably genetically transformed arthropod individuals, or more preferably, genetically transformed insect individuals. New transposons, such as piggyBac, can be used to transform various insects (Handler A.M. Insect Biochem. Molec. Biol. 31:118-128., 2001; Atkinson, P.W. & James, A.A. Adv. Genet. 47:49-86., 2002; Tamura, T. Nature Biotechnol. 18:81-84, 2000).

For example, by producing a transformed insect introduced with a DNA in which a DNA encoding a juvenile hormone acid methyltransferase of the present invention is bound downstream of a promoter which can be induced at an arbitrary time (such as a heat shock promoter or a tetracycline-responsive promoter), expression of the DNA encoding the juvenile hormone acid methyltransferase can be induced at will. Therefore, extra-molting can be induced in final instar larvae where juvenile hormone synthesis is ordinarily stopped, by continuously synthesizing and secreting juvenile hormone.

Further, for example, by producing a transformed insect introduced with a DNA in which a DNA encoding an antisense, sense, or double-stranded RNA to a DNA encoding a juvenile hormone acid methyltransferase of the present invention is bound downstream of a promoter which can be induced at an arbitrary time (such as a heat shock promoter or a tetracycline-responsive promoter), expression of the DNA encoding juvenile hormone acid methyltransferase can be stopped at will, thereby decreasing the concentration of juvenile hormone in the insect body at will. For example, by inhibiting expression of juvenile hormone acid methyltransferase in the larva stage before the final instar of the transformed insect, juvenile hormone synthesis can be inhibited, and early metamorphosis can be induced at will.

In addition, the present invention provides antibodies that bind to the protein of the present invention. The antibodies of the present invention comprise monoclonal antibodies, polyclonal antibodies, antibody mutants, and fragments thereof.

These antibodies can be prepared by methods known to those skilled in the art. Polyclonal antibodies can be obtained, for example, by the following process: A small animal (such as a rabbit) is immunized with a juvenile hormone acid methyltransferase of *Bombyx mori, Drosophila melanogaster, Anopheles gambiae, Spodoptera litura,* or *Helicoverpa armigera,* or a recombinant protein expressed in a microorganism (such as *Escherichia coli)* as a fusion protein with GST, or a partial peptide thereof, and the serum is collected. The serum is purified and prepared, for example, by ammonium sulfate precipitation, protein A or protein G column chromatography, DEAE ion-exchange chromatography, or affinity column coupled with the juvenile hormone acid methyltransferase of *Bombyx mori, Drosophila melanogaster, Anopheles gambiae, Spodoptera litura,* or synthetic peptides. Monoclonal antibodies can be obtained, for example, by the following process: A small animal (such as a mouse) is immunized with a juvenile hormone acid methyltransferase of *Bombyx mori, Drosophila melanogaster, Anopheles gambiae, Spodoptera litura,* or *Helicoverpa armigera,* or a partial peptide thereof, then their spleens are extirpated and ground to separate the cells. The cells are fused with mouse myeloma cells using a reagent such as polyethylene glycol, thus obtaining fused cells (hybridoma) from which are selected clones for producing antibodies which bind to a juvenile hormone acid methyltransferase of *Bombyx mori, Drosophila melanogaster, Anopheles gambiae, Spodoptera litura,* or *Helicoverpa armigera.* The hybridomas thus obtained are then implanted intraperitoneally to a mouse, ascites are then recovered, and monoclonal antibody is obtained. Monoclonal antibodies thus obtained are purified by, for example, ammonium sulfate precipitation, protein A or protein G column chromatography, DEAE ion-exchange chromatography, or by an affinity column coupled with the juvenile hormone acid methyltransferase of *Bombyx mori, Drosophila melanogaster, Anopheles gambiae, Spodoptera litura,* or a synthetic peptide.

In the present invention, "mutant antibody" refers to antibodies with amino acid sequence variations, in which one or more amino acid residues are altered. A "mutant antibody" of the present invention comprises variously altered amino acid variants, as long as they have the same binding specificity as the original antibody. Such mutants have less than 100% homology or similarity to an amino acid sequence that has at least 75%, more preferably at least 80%, even more preferably at least 85%, still more preferably at least 90%, and most preferably at least 95% amino acid sequence homology or similarity to the amino acid sequence of the variable domain of a heavy chain or light chain of an antibody.

The present invention also provides oligonucleotides comprising at least 15 nucleotides that are complementary to a DNA of the present invention, or to a complementary strand thereof.

A "complementary strand" herein refers to one strand of a double strand DNA comprising A:T (or A:U for RNA) and G:C base pairs, when viewed against the other strand. Furthermore, "complementary" means not only nucleotide sequences completely complementary to a continuous nucleotide sequence of at least 15 nucleotides, but also those with a homology of at least 70%, preferably at least 80%, more preferably 90%, and most preferably 95% or more at the nucleotide sequence level. Homology can be determined using an algorithm described herein. The term "oligonucleotide" also comprises polynucleotides.

The oligonucleotides of the present invention can be used as probes and primers for the detection and amplification of DNAs encoding the proteins of the present invention, and probes and primers for detecting the expression of the DNAs. Moreover, the oligonucleotides of the present invention may be used in the form of a DNA-array substrate.

When used as primers, the oligonucleotides may usually comprise 15 to 100 bp, preferably 17 to 30 bp. The primer is not limited, as long as it can amplify at least a portion of a DNA of the present invention, or a complementary strand thereof. In addition, when used as a primer, the 3' side of the oligonucleotide may be designed to be complementary, and a restriction enzyme recognition sequence or tag may be attached to the 5' side of the same.

Moreover, when an above-described oligonucleotide is used as a probe, the probe is not particularly limited as long as it hybridizes specifically to at least a portion of a DNA of the present invention, or a complementary strand thereof. The probe may be also a synthetic oligonucleotide, which usually comprises at least 15 bp or more.

When the oligonucleotides of the present invention are used as probes, they are preferably appropriately labeled. Examples of labeling methods comprise methods in which T4 polynucleotide kinase is used to label the 5' end of an oligonucleotide by ³²P phosphorylation, or methods in which DNA polymerases such as Klenow enzyme, and primers such as random hexamer oligonucleotides, are used to incorporate nucleotides labeled with isotopes such as ³²P, fluorescent dyes, biotin, and such (random priming methods and the like) into the oligonucleotide substrate.

The oligonucleotides of the present invention may be produced by, for example, a commercially available oligonucleotide synthesizer. Probes may also be prepared as double-stranded DNA fragments by restriction enzyme treatment and such.

The present inventors isolated juvenile hormone acid methyltransferase genes. *Escherichia coli* or yeast was transformed with vector DNAs, such as plasmids incorporating these genes, to synthesize large amounts of recombinant protein, which could then be used to easily and cheaply screen for compounds that bind juvenile hormone acid methyltransferase and compounds which regulate activity or expression of juvenile hormone acid methyltransferase. Thus, the present invention provides methods of screening for such compounds. The compounds obtained by the screening methods of the present invention can be used as agents for regulating the molting/metamorphosis, reproduction, diapause, blastogenesis, behavior, polymorphism, or life span of arthropods (preferably insects). Further, the screening methods can be used to provide compounds applicable as insect growth regulators (Shokubutsu Boueki Kouza (Plant Protection Course), Ver. 3, Pests/Harmful Animals, Japan Plant Protection Association, p132) or Nouyaku Handbook (Agricultural Chemical Handbook), Ver. 2001, Japan Plant Protection Association, 2001 Ver. 11, p127)). The term "Insect growth regulator (IGR)" means an agent which acts specifically on insects, disturbing their molting/metamorphosis to exhibit insecticidal effects, or kills their egg or sterilizes adult female insects to reduce the head count of succeeding generations. See the above documents for more detailed definitions.

One embodiment of a screening method of the present invention relates to screening for compounds that bind a protein of the present invention. Such methods comprises the steps of contacting a test compound with a protein of the present invention, detecting binding of the protein and the test compound, and selecting a compound which binds to the protein of the present invention.

The proteins of the present invention for use in screening may be recombinant or naturally occurring proteins, and are preferably recombinant proteins. Alternatively, the proteins of the present invention for use in screening may be partial peptides.

The test compounds are not particularly limited, and comprise cell extracts, cell culture supernatants, fermented microorganism products, marine organism extracts, plant extracts, purified or crude proteins, non-peptide compounds, synthetic low molecular weight compounds, and natural compounds. The proteins of the present invention for contacting with test compounds may be in the form of purified proteins, soluble proteins, proteins bound to a carrier, or fusion proteins with other proteins.

Various methods known to those skilled in the art may be used as methods of screening for proteins that bind to the proteins of the present invention, using a protein of the present invention. Such screening can be carried out, for example, using immunoprecipitation methods. Specifically, the methods can be carried out as follows: DNAs encoding the proteins of this invention are expressed by inserting them into vectors for foreign gene expression, such as pSV2neo, pcDNA I, and pCD8; and then expressing the gene in animal cells, etc. Any generally used promoters may be employed for the expression, including the SV40 early promoter (Rigby In Williamson (ed.), Genetic Engineering, Vol. 3. Academic Press, London, p.83-141 (1982)), etc.

Introduction of a foreign gene into animal cells for expression therein can be performed by any of the following methods, including electroporation methods (Chu, G. et al., Nucl. Acid Res. 15, 1311-1326 (1987)), calcium phosphate methods (Chen, C. and Okayama, H. Mol. Cell. Biol. 7, 2745-2752 (1987)), DEAE dextran methods (Lopata, M. A. et al. Nucl. Acids Res. 12, 5707-5717 (1984); Sussman, D. J. and Milman, G. Mol. Cell. Biol. 4, 1642-1643 (1985)), lipofectin methods (Derijard, B. Cell. 7, 1025-1037 (1994); Lamb, B. T. et al. Nature Genetics 5, 22-30 (1993)), Rabindran, S. K. et al. Science 259, 230-234 (1993)), etc.

The proteins of this invention can be expressed as fusion proteins that comprise a recognition site of a monoclonal antibody whose specificity has been established, by introducing the recognition site (epitope) into the N- or C-terminus of a protein of this invention. For this purpose, a commercial epitope-antibody system can be utilized (Jikken Igaku (Experimental Medicine) 13, 85-90 (1995)). Vectors that express fusion proteins via a multi-cloning site with β-galactosidase, maltose-binding protein, glutathione S-transferase, green fluorescence protein (GFP), and such are commercially available.

To minimize changes to the properties of the proteins of this invention which may be caused by fusion protein formation, methods for preparing fusion proteins have been reported that involve introducing only a small epitope portion, comprising a few to dozens of amino acid residues. For example, the epitopes of polyhistidine (His-tag), influenza hemagglutinin (HA), human c-myc, FLAG, Vesicular stomatitis virus glycoprotein (VSV-GP), T7 gene 10 protein (T7-tag), human herpes simplex virus glycoprotein (HSV-tag), E-tag (epitope on the monoclonal phage), and such, and monoclonal antibodies that recognize these epitopes, can be utilized as epitope-antibody systems for screening proteins that bind to the proteins of this invention (Jikken Igaku (Experimental Medicine) 13, 85-90 (1995)).

For immunoprecipitation, immune complexes are formed by adding these antibodies to cell lysates prepared using suitable surfactants. These immune complexes comprise a protein of this invention, a protein that binds to this protein, and an antibody. In addition to using antibodies against the above-described epitopes to perform immunoprecipitation, antibodies against the proteins of this invention can also be used. An antibody against a protein of this invention can be prepared by, for example, inserting a gene encoding a protein of this invention into an appropriate expression vector of *E. coli,* purifying the protein thus expressed in the bacterium, and immunizing rabbits, mice, rats, goats, chickens, and such, with the purified protein. The antibodies can also be prepared by immunizing the above-described animals with partial peptides of the proteins of this invention.

When the antibody is a murine IgG antibody, immune complexes can be precipitated using, for example, Protein A Sepharose and Protein G Sepharose. In addition, when the proteins of this invention are prepared as fusion proteins with the epitope of, for example, GST or such, the immune complexes can be formed using substances that specifically bind to these epitopes, such as glutathione-Sepharose 4B, giving the same result as when using an antibody for a protein of this invention.

In general, immunoprecipitation may be carried out according to, or in line with, the methods described in the literature (Harlow, E. and Lane, D.: Antibodies, pp.511-552, Cold Spring Harbor Laboratory publications, New York (1988)).

SDS-PAGE is generally used to analyze immunoprecipitated proteins. By using a gel of an appropriate concentration, bound proteins can be analyzed based on their molecular weight. Generally, in such cases the proteins that have bound to the proteins of this invention can hardly be detected by usual protein staining methods, such as Coomassie staining and silver staining. However, detection sensitivity can be improved by culturing the cells in a medium comprising radio isotope-labeled ³⁵S-methionine and ³⁵S-cysteine to label proteins inside the cells, and detecting the labeled proteins. After determining the molecular weight of a desired protein, the protein can be directly purified from an SDS-polyacrylamide gel, and then sequenced.

Proteins that bind to the proteins of the present invention by using these proteins may be isolated by West-Western blotting (Skolnik E.Y et al. (1991) Cell 65: 83-90). Specifically, using phage vectors (λgt11, ZAP, etc.), a cDNA library is constructed from cells or tissues expected to express proteins that bind to a protein of the present invention. This cDNA library is then expressed on an LB-agarose plate and transferred to a filter membrane. The filter membrane is reacted with purified and labeled proteins of the invention. Plaques expressing proteins that bind to a protein of the invention can be identified by detection of the label. The proteins of the invention may be labeled by methods that utilize binding between biotin and avidin, or methods utilizing antibodies that specifically bind to a protein of the present invention, or a protein (such as GST) that is fused to a protein of the present invention. Methods using radioisotopes or fluorescence and such may also be used.

Alternatively, according to another embodiment of the screening methods of the present invention, a two-hybrid system that utilizes cells may be used (Fields S. and Sternglanz R. (1994) Trends Genet. 10: 286-292; Dalton S. and Treisman R. (1992) "Characterization of SAP-1, a protein recruited by serum response factor to the c-fos serum response element." Cell 68: 597-612; "MATCHMAKER Two-Hybrid System", "Mammalian MATCHMAKER Two-Hybrid Assay Kit", "MATCHMAKER One-Hybrid System" (products of Clontech); "HybriZAP Two-Hybrid Vector System" (Stratagene)).

A two-hybrid system can be used as follows: (1) a protein of the present invention or partial peptide thereof is fused to a SRF DNA binding region or GAL4 DNA binding region, and expressed in yeast cells; (2) a cDNA library, which expresses proteins as fusion proteins with VP16 or GAL4 transcription activating regions, is prepared from cells expected to express the proteins binding to the protein of the present invention; (3) the library is introduced to the above-mentioned yeast cells; and (4) library-derived cDNAs are isolated from the positive clones detected (positive clones can be confirmed by the activation of reporter genes on binding of the present protein and the binding protein expressed in the yeast cell). The proteins encoded by the cDNAs can be obtained by introducing and expressing the isolated cDNAs in *E*. *coli.* Thus, proteins that bind to the present proteins, or genes thereof, can be prepared.

In addition to the HIS3 gene, reporter genes for use in two-hybrid systems include, for example, the Ade2 gene, LacZ gene, CAT gene, luciferase gene, PAI-1 (Plasminogen activator inhibitor type1) gene, and such, but are not restricted thereto. Screening using a two-hybrid system can be also carried out using mammalian cells, in addition to yeast.

Alternatively, compounds binding to the proteins of the present invention can be screened using affinity chromatography. For example, the proteins of the invention are immobilized to an affinity column carrier, and a test sample predicted to express a protein that binds to a protein of the invention is applied to the column. The test samples used herein may be cell extracts, cell lysates, and so on. After loading the test samples, the column is washed, and proteins that bind to the proteins of the invention can be obtained.

The DNAs that encode the proteins may be obtained by analyzing the amino acid sequence of the obtained proteins, synthesizing oligo-DNAs based on this sequence information, and screening a cDNA library using these DNAs as probes.

As methods for isolating compounds (not limited to proteins) which bind to the proteins of the present invention, those skilled in the art know, for example, methods wherein synthetic compounds, natural product banks, or random phage peptide display libraries are acted upon an immobilized protein of the present invention to screen for compounds which bind the protein, or screening methods using high throughput combinatorial chemistry technology.

In the present invention, the bound compounds can be detected or determined by, for example, labels tagged to test compounds. Labels comprise fluorescent labels and radioactive labels. Biosensors using surface plasmon resonance phenomenon can also be used. Biosensors using surface plasmon resonance phenomenon use a tiny amount of protein and do not require labeling, and can observe real time interactions between a protein of the present invention and a test compound using surface plasmon resonance signals (for example, BIAcore, produced by Pharmacia). Therefore, biosensors such as BIAcore can be used to evaluate binding of a protein of the present invention to a test compound.

Compounds selected using the above-mentioned screening methods include compounds which regulate the activity of a protein of the present invention, and compounds which regulate the expression of a DNA of the present invention. In the present invention, compounds selected using an above-mentioned screening method can also be used as test compounds for screening methods described later.

Another embodiment of the screening methods of the present invention relates to screening for compounds that regulate the activity of a protein of the present invention. In the first step of such methods, a test compound is contacted with a protein of the present invention. The proteins of the present invention can be, for example, purified, expressed in cells, expressed in cell extract solutions, and so on, and their state is not particularly limited.

Cells in which a protein of the present invention is expressed comprise cells in which an endogenous protein of the present invention is expressed, or cells in which an exogenous protein of the present invention is expressed. The above-mentioned cells, in which an exogenous protein of the present invention is expressed, can be prepared by introducing a vector comprising a DNA encoding a protein of the present invention into the cell. The vector can be introduced to a cell by methods ordinary to one skilled in the art. Alternatively, the above-mentioned cells that comprise an exogenous protein of the present invention can be prepared by inserting a DNA encoding a protein of the present invention into a chromosome by genetic transfer methods using homologous recombination. The kind of organism from which cells inserted with an exogenous protein of the present invention are derived is not particularly limited, and may be an animal for which the technology for expressing foreign proteins in cell is established.

The cell extract solutions in which a protein of the present invention is expressed comprise, for example, solutions wherein a vector comprising a DNA encoding a protein of the present invention is added to a cell extract solution comprising an *in vitro* transcription/translation system. The *in vitro* transcription/translation system is not particularly limited, and a commercially available *in vitro* transcription/translation kit may be used.

In the present invention, "contact" is carried out depending on the state of the proteins of the present invention. For example, when a protein of the present invention is purified, contact is carried out by adding a test compound to the purified sample. When the protein is expressed in a cell or in a cell extract solution, contact is carried out by adding a test compound to the cell culture solution or to the cell extract solution, respectively. If the test compound is a protein, the contact can be carried out, for example, by introducing a vector comprising a DNA encoding the protein into a cell expressing a protein of the present invention. The contact can also be carried out by adding the vector to a cell extract expressing the protein of the present invention. Alternatively, two-hybrid methods using, for example, a yeast or an animal cell may be used.

In another embodiment, the activity of the above-mentioned proteins of the present invention is then determined. Compounds that increase or decrease the activity of a protein of the present invention are selected by comparison with cases where test compound was not administered.

The activity of the proteins of the present invention comprises juvenile hormone production activity. As described in the Examples, when juvenile hormone acid such as JH I acid, JH II acid, JH III acid, and farnesoic acid are substrates, the production of their respective corresponding active juvenile hormones, that is JH I, JH II, JH III, and methyl farnesoate in a reaction solution, can be confirmed to determine the activity a protein of the present invention. In addition to HPLC analysis as described in the Examples, GC, GC/MS, LC/MS, RIA, and bioassays can be used to determine the activity of proteins of the present invention (Tetsuya Otaki: Seiri Kassei Busshitsu no Bioassay (Bioassay of physiologically active substances), Kodansha, p.373-380, 1984; Rembold, H and Lackner, B.: J. Chromatogr. 323:355-361, 1985). Alternatively, to determine the activity of the protein of the present invention, the amount by which S-adenosyl methionine is reduced, or the amount of S-adenosyl homocysteine produced in a reaction solution containing juvenile hormone acid can also be determined by various methods. For example, adenosyl homocysteine hydrolase (AdoHcy hydrolase) and adenosine deaminase are made to coexist in a reaction solution, and the S-adenosyl homocysteine thus produced is finally changed to inosine. The decrease in absorption at 265 nm caused by the change from S-adenosyl methionine to inosine can be measured to determine this activity (Ogawa H. et al., 1988, Proc. Natl. Acad. Sci. U.S.A. 85, 694-698).

The above-mentioned screening methods can be carried out, for example, by the following procedure: A test compound is first mixed in a reaction solution containing JH I acid, JH II acid, JH III acid, or farnesoic acid to carry out the enzyme reaction. After the reaction, the amount of juvenile hormone produced is compared with that produced without test compound. The compositions described in the Examples can be used as reaction solutions containing JH I acid, JH II acid, JH III acid or farnesoic acid.

Another embodiment of the screening methods of the present invention relates to screening for compounds that regulate the expression levels of genes encoding the proteins of the present invention. These methods provide cells or cell extract solutions comprising a DNA in which a reporter gene is functionally bound downstream of a promoter domain of a gene encoding a protein of the present invention. Herein, "functionally bound" means the promoter region of the gene encoding the protein of the present invention and the reporter gene are bound such that a transcription factor binding to the promoter region can induce reporter gene expression. Accordingly, if a reporter gene is bound to another gene to form a fusion protein with the other gene product, this is also included in the meaning of "functionally bound" as mentioned above, as long as the transcription factor binds to the promoter region to induce expression of the fusion protein.

The above-mentioned reporter genes are not particularly limited, as long as their expression is detectable. The reporter genes comprise the CAT gene, lacZ gene, luciferase gene, β-glucuronidase gene (GUS), and GFP gene, which are in general use by those skilled in the art. The above-mentioned reporter genes also comprise DNAs encoding proteins of the present invention.

In another embodiment, test compounds are contacted with an above-mentioned cell or cell extract solution, and the expression level of the above-mentioned reporter gene in the cell or cell extract solution is then determined. The expression level of a reporter gene can be determined using methods known to those skilled in the art, depending on the kind of the reporter gene used. For example, if the reporter gene is CAT gene, the acetylation of chloramphenicol caused by the gene product can be detected to determine reporter gene expression level. If the reporter gene is lacZ gene, the coloration of a pigment compound caused by the enzyme action of the gene expression product can be detected to determine reporter gene expression level. If the reporter gene is luciferase, the fluorescence of a fluorescent compound caused by the enzyme action of the gene expression product can be detected to determine reporter gene expression level. If the reporter gene is β-glucuronidase gene (GUS), the luminescence of Glucuron (ICN Co.) or the coloration of 5-bromo-4-chloro-3-indolyl-β-glucuronide (X-Gluc) caused by the enzyme action of the gene expression product can be detected to determine reporter gene expression level. If the reporter gene is GFP, the fluorescence of the GFP protein can be detected to determine reporter gene expression level.

If a gene encoding a protein of the present invention is used as a reporter, the expression level of that gene can be determined by methods known to those skilled in the art. For example, the mRNA of the gene is extracted by conventional methods, then the mRNA can be used as a template for Northern hybridization method or RT-PCR to determine the level of gene transcription. Further, DNA array techniques can be used to determine the expression level of the gene.

The translation level of a gene encoding a protein of the present invention, encoded by a gene coding a protein of the present invention, can be determined by recovering a fraction comprising the protein of the present invention by conventional methods, and then detecting the expression of the protein of the present invention by electrophoresis methods such as SDS-PAGE. Alternatively, the translation level of the gene can be determined by methods such as Western blotting using an antibody against the protein of the present invention, to detect expression of the protein of the present invention. In another embodiment, compounds that increase or decrease reporter gene expression level compared to cases without test compound are selected.

In another embodiment of the screening methods of the present invention, a test compound is first contacted with an insect individual or cultured tissue. Cultured corpora allata tissue is suitable as the tissue culture. In another embodiment, the expression level of a gene encoding a protein of the present invention is then determined in the insect individual or cultured tissue. Specifically, a test compound is contacted with the insect individual or cultured tissue, and after a certain time RNA is extracted to determine the amount of gene expression using quantitative PCR, DNA microarray, Northern blotting, and the like. In another embodiment, compounds that increase or decrease the expression level of the gene compared to cases without test compound are selected.

### Brief Description of the Drawings

Fig. 1 is a diagram showing an enzyme reaction between enzymes of the present invention and juvenile hormone and its precursor acid, found in insects and crustaceans. JHAMT indicates the juvenile hormone acid methyltransferase, AdoMet indicates S-adenosyl methionine, and AdoHcy indicates S-adenosyl homocysteine. R₁ and R₂ in the juvenile hormone acid are: R₁= CH₃ and R₂= CH₃ in JH I acid; R₁= CH₃ and R₂= H in JH II acid; and R₁= H and R₂= H in JH III acid. R₁ and R₂ in the juvenile hormone are: R₁= CH₃ and R₂= CH₃ in JH I; R₁= CH₃ and R₂= H in JH II; and R₁= H and R₂= H in JH III.

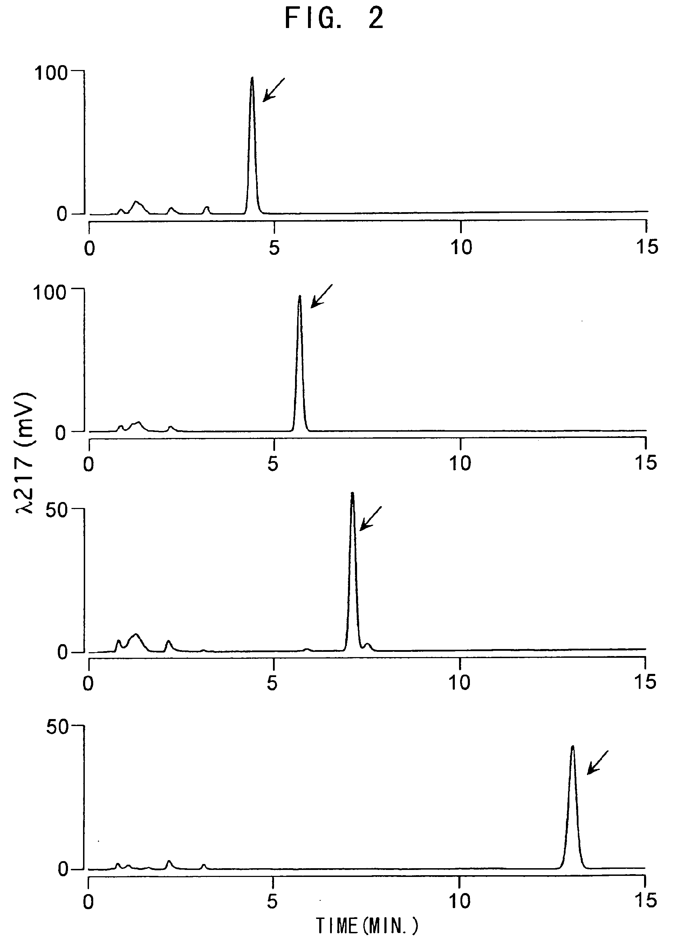

Fig. 2 is a diagram showing the detection of juvenile hormone by reverse phase HPLC. The detection of JH III, JH II, JH I, and methyl farnesoate are shown in order from the top.

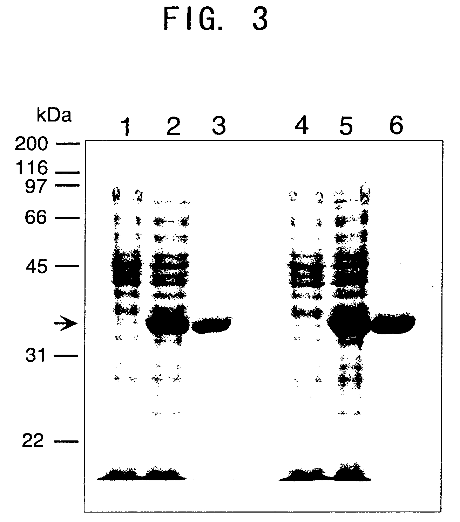

Fig. 3 is a photograph showing SDS electrophoresis of the recombinant enzyme proteins of the present invention. Lanes 1 and 4 show the soluble fractions (controls) of crude extracts from *Escherichia coli* transformed with pET28a plasmid. Lane 2 shows a crude extract from *Escherichia coli* transformed with pET28a plasmid comprising the juvenile hormone acid methyltransferase gene from *Bombyx mori.* Lane 3 shows a soluble fraction of the purified juvenile hormone acid methyltransferase protein from *Bombyx mori.* Lane 5 shows a crude extract from *Escherichia coli* transformed with pET28a plasmid comprising the juvenile hormone acid methyltransferase gene from *Drosophila melanogaster.* Lane 6 shows the purified juvenile hormone acid methyltransferase protein from *Drosophila melanogaster.* The arrow shows a recombinant juvenile hormone acid methyltransferase protein.

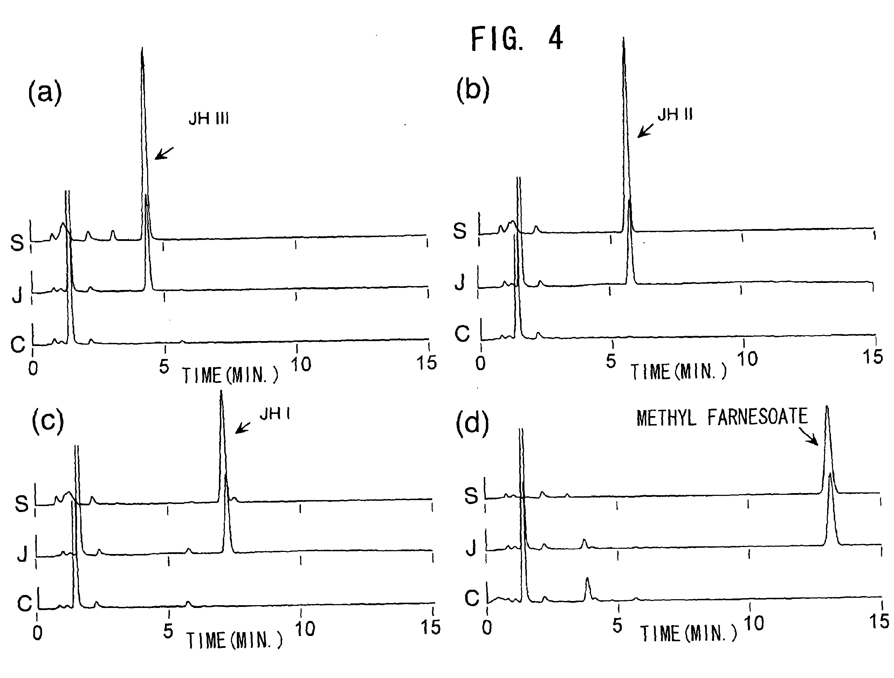

Fig. 4 is a diagram showing the enzyme activities of the recombinant proteins of the present invention. "S" indicates a standard juvenile hormone substance. "J" indicates a reaction product obtained by the action of recombinant juvenile hormone acid methyltransferase from *Bombyx mori* on either one of JH III acid (a), JH II acid (b), JH III acid (c), or farnesoic acid (d) with S-adenosyl methionine as a substrate. Juvenile hormones corresponding to each substrate, that is, JH III (a), JH II (b), JH III (c) or methyl farnesoate (d), are confirmed to be produced. "C" is a reaction product of the case where no enzyme is added to the reaction solution comprising the same substrates as in "J". No juvenile hormone is produced.
Fig. 5 shows the results of multiple alignment of the amino acid sequences of juvenile hormone acid methyltransferases derived from *Bombyx mori, Drosophila melanogaster, Anopheles gambiae, Spodoptera litura,* and *Helicoverpa armigera.* Sl indicates the juvenile hormone acid methyltransferase derived from *Spodoptera litura,* Ag from *Anopheles gambiae,* Ha from *Helicoverpa armigera,* Bm from *Bombyx mori,* and Dm from *Drosophila melanogaster.*

### Best Mode for Carrying Out the Invention

The present invention will be described more specifically with reference to Examples below, but is not limited to these Examples.

### (1) Detection of juvenile hormones by HPLC

A reverse phase column (CAPCELL PAK C18 UG80 S-5, 3x 150 mm, Shiseido) was attached to an HPLC apparatus (JASCO), and juvenile hormones were detected by measuring absorbance at 217 nm using an ultraviolet detector. Samples were flowed at a rate of 0.5 ml/min, at a column retention temperature of 40°C, for 20 minutes, in 60% acetonitrile or 80% acetonitrile.

### (Example 1) Cloning the juvenile hormone acid methyltransferase gene of Bombyx mori

The corpora allata is a minute tissue (diameter about 0.1 mm even in a final instar larva of *Bombyx mori).* Hence, in order to obtain from the corpora allata an amount of RNA necessary for cloning the juvenile hormone acid methyltransferase, many individuals must be dissected to collect their corpora allata. *Bombyx mori* is large and easy to dissect, with a large corpora allata compared to pests such as *Drosophila melanogaster, Anopheles gambiae, Spodoptera litura,* and *Helicoverpa armigera.* Further, large-scale breeding methods using artificial feed are established, and thus the amount of larvae of uniform growth stage necessary for the experiment may be easily gathered. The present inventors then attempted to clone the juvenile hormone acid methyltransferase gene of *Bombyx mori.*

RNAs were extracted from the corpora allata of *Bombyx mori* larvae classified into 24 steps of growth stages, from the early stage of 4th instar to pupa (about 40 per stage × 24 stages = about 1,000 dissected). These RNAs were used as templates for reverse transcription reactions using the FP1 primer (SEQ ID NO: 11) with its 5' end labeled with Rhodamine, thereby producing 24 kinds of cDNA. These cDNAs were used as templates for PCR reactions using fluorescent primer FP 1 and a decamer of arbitrary sequence (24 types were used), thereby amplifying fluorescence-labeled cDNAs. The amplified cDNAs were separated by a modified acrylamide gel, and scanned using a fluorescent image analyzer (FMBIO II, Takara Co.) to detect the cDNA bands. The present inventors examined about 400 cDNA bands exhibiting various growth-specific expression patterns. As a result, for the cDNA amplified using a combination of the primer FP1 and the primer UP1 (SEQ ID NO: 12), a single fluorescent band was discovered that was expressed continuously during 4th instar larva stage, but disappeared after the 3rd day of 5th instar larva stage. A cDNA fragment was extracted from this fluorescent band and its nucleotide sequence was determined.

A primer was designed based on this determined nucleotide sequence, and then 5' RACE and 3' RACE (a kind of modified RT-PCR) were performed using a SMART RACE cDNAAmplification Kit (CLONTECH CO.) to clone the full length cDNA. For the 3'RACE, BM3R primer (SEQ ID NO: 13) was used to amplify the region from 1880 to 2890 of the entire nucleotide sequence, and for the 5'RACE, BM5R primer (SEQ ID NO: 14) was used to amplify the region from 1 to 1904 of the entire nucleotide sequence, thereby determining the nucleotide sequence (SEQ ID NO: 1).

As described above, the full length cDNA for the juvenile hormone acid methyltransferase of *Bombyx mori* was determined by overlapping the regions cloned by 5' RACE and 3' RACE fluorescent mRNA differential display method.

### (Example 2) Production of an expression vector incorporating the juvenile hormone acid methyltransferase gene

The cDNA from the corpora allata of *Bombyx mori* 4th instar larvae was used as a template for PCR using the BMJF primer (SEQ ID NO: 15) and BMJR primer (SEQ ID NO: 16) to amplify a region comprising the open reading frame for the gene isolated by the method of Example 1. The amplified DNA was cleaved with restriction enzymes *Nde*I and *Bam*HI*,* followed by cloning at the *Nde*I*-Bam*HI site of the *Escherichia coli* expression vector pET-28a(+) for producing histidine tag fusion proteins (Novagen Co.).

### (Example 3) Preparation of recombinant juvenile hormone acid methyltransferase protein

The produced plasmid was introduced in to *Escherichia coli* BL21 (DE3) by electroporation to obtain a transformed *Escherichia coli* strain. The transformed *Escherichia coli* strain was cultured in LB medium (5 ml) comprising 50 µg/ml Kanamycin at 37°C overnight while shaking. Then 0.2 ml of the culture solution was added to 200 ml of liquid medium, and this was cultured while shaking at 20°C for 24 hours. The proliferated bacteria were recovered from the culture solution by centrifugation (3,500 rpm, 10 minutes, 4°C), and frozen at -30°C. The frozen bacteria were liquified at room temperature, and then 5 ml of buffer (50 mM Tris-Cl, pH 7.5) per 50 ml of culture solution was added to suspend the bacteria. This was followed by ultrasonic disintegration while cool. After centrifugation (14,000 rpm, 15 minutes, 4°C), the supernatant was collected, and filtered through a 0.45 µm filter, then the recombinant juvenile hormone acid methyltransferase was purified using a histidine tag fusion protein purification kit (HiTrap Kit, Amersham Co.). The eluted recombinant protein was applied to a desalting column (PD-10, Amersham Co.) to remove imidazole, then stored at -30°C as a 25 mM Tris-Cl buffer, pH 7.5, 50% glycerol solution. The yield from 50 ml of the culture solution was about 4 mg (total liquid amount: 7 ml) (Fig. 3).

### (Example 4) Juvenile hormone synthesis by recombinant juvenile hormone acid methyltransferase.

100 µg of a juvenile hormone acid (either JH I acid, JH II acid, JH III acid, or farnesoic acid) dissolved in toluene was transferred into a silicone-treated glass test tube (10 x 150 mm) using a glass capillary, and the solvent was removed under a nitrogen stream. 800 µl of buffer (50 mM Tris-Cl, pH 7.5) and 100 µl of 10 mM S-adenosyl methionine solution were added thereto and mixed sufficiently, then left to stand in a 25°C thermostat water bath for five minutes. 100 µl of the recombinant protein solution obtained by the method of Example 3 was added thereto to initiate enzyme reaction. After ten minutes, 250 µl of reaction termination solution (methanol : water : concentrated aqueous ammonia = 10 : 9 : 1) was added to terminate the enzyme reaction 1 ml of isooctane was added and mixed sufficiently, then the mixture was centrifuged (2,000 rpm, 15 minutes, room temperature) to separate the organic solvent layer and the aqueous layer. 100 µl of the organic solvent layer was transferred into a small vial using a glass capillary, and then the solvent was removed under a nitrogen stream to obtain a residue, which was dissolved in 100 µl of acetonitrile to analyze the synthesized juvenile hormone by a method using reverse phase HPLC. As a result, the active juvenile hormones corresponding to when JH I acid, JH II acid, JH III acid, and farnesoic acid were used as the substrate, that is, JH I, JH II, JH III, and methyl farnesoate respectively, were confirmed to be produced in the reaction solutions (Fig. 4). The molecular activities of the recombinant *Bombyx mori* JHAMT against JH I acid, JH II acid, JH III acid, and farnesoic acid were determined in conditions where the buffer was Tris-HCL (50 mM, pH 7.5), the reaction temperature was 25°C, and the substrates were1mM of S-adenosyl methionine and 100 µM of each JH acid. These activities (the number of substrate molecules changed to reaction product by one molecule of the enzyme in one minute) were 1.80 ± 0.32,1.65 ± 0.23, 0.79 ± 0.06, and 0.48 ± 0.07 (mean ± standard deviation, n=3), respectively. When a saturated straight chain fatty acid or an unsaturated straight chain fatty acid was used as a substrate, methyl esterification by the enzyme was not observed. Therefore, the enzyme reaction was substrate-specific to juvenile hormone acid.

### (Example 5) Cloning of the juvenile hormone acid methyltransferase genes of Drosophila melanogaster and Anopheles gambiae.

The amino acid sequence of the juvenile hormone acid methyltransferase from *Bombyx* *mori,* as shown in SEQ ID NO: 2, was used to search the whole genome database of *Drosophila melanogaster* and *Anopheles gambiae* which is an important malarial vector. As a result, a DNA encoding the amino acid sequence shown in SEQ ID NO 4 (SEQ ID NO: 3), which showed 38% identity to the amino acid sequence of the juvenile hormone acid methyltransferase of *Bombyx mori* (SEQ ID NO: 2), was found in the *Drosophila melanogaster* genome.

cDNAs synthesized by reverse transcription of RNA extracted from a whole adult *Drosophila melanogaster* were used as a template for PCR using the DMJF primer (SEQ ID NO: 17) and DMJR primer (SEQ ID NO: 18) to amplify the coding region of the juvenile hormone acid methyltransferase gene from *Drosophila melanogaster.* The same method as in Example 2 was used to produce an expression vector, which was then used to produce a recombinant protein by the same method as in Example 3. The activity of the recombinant protein was determined by the same method as in Example 4, confirming that the protein encoded by the gene had juvenile hormone acid methyltransferase activity. The molecular activities of the recombinant *Drosophila melanogaster* JHAMT against JH I acid, JH II acid, JH III acid, and farnesoic acid were determined under conditions where the buffer was Tris-HCL (50 mM, pH 7.5), the reaction temperature was 25°C, and the substrates were 1 mM of S-adenosyl methionine and 100 µM of each JH acid. These activities were determined to be 2.67 ± 0.44, 2.07 ± 0.26, 1.76 ± 0.13, and 2.3 ± 0.51 (mean ± standard deviation, n=3), respectively.

Using a data base, from the entire genome sequence of *Anopheles gambiae,* the present inventors found a gene (SEQ ID NO: 5) that encodes the amino acid sequence encoding the juvenile hormone acid methyltransferase from *Anopheles gambiae,* SEQ ID NO: 6, which was 38% identical to the amino acid sequence of the juvenile hormone acid methyltransferase of *Bombyx mori* and 47% to that of *Drosophila melanogaster.* Because juvenile hormone acid methyltransferase is essential for the survival of insects, and its gene certainly exists in the genome of *Anopheles gambiae,* and further, since no gene having another similar sequence exists in the entire genome of *Anopheles gambiae,* this gene clearly encodes the enzyme of *Anopheles gambiae.*

### (Example 6) Cloning of the juvenile hormone acid methyltransferase genes of Lepidoptera pests

The juvenile hormone acid methyltransferase genes of *Spodoptera litura* and *Helicoverpa armigera,* important pests that cause damage to many crops, were cloned as described below.

The amino acid sequence of the juvenile hormone acid methyltransferase of *Bombyx mori* (SEQ ID NO: 2) and the amino acid sequence of the juvenile hormone acid methyltransferase of *Drosophila melanogaster* (SEQ ID NO: 4) were compared. Based on well-conserved regions of both amino acid sequences , two degenerate primers were prepared: the DGJF primer (SEQ ID NO: 19; encoding the amino acid sequence of MVKYANKH) as a forward primer and the DGJR primer (SEQ ID NO: 20; encoding the amino acid sequence of FDHVFSFY) as a reverse primer. These primers were used to synthesize cDNAs by reverse transcription of RNAs extracted from the corpora allata of penultimate instar larvae of *Spodoptera litura* and *Helicoverpa armigera.* The cDNAs were used as templates for their respective PCRs, providing DNA fragments of about 120bp. Their nucleotide sequences were then determined, and specific primers were designed based on these sequences. Then 5' RACE and 3' RACE, a kind of modified RT-PCR, were carried out using SMART RACE cDNA Amplification Kit (CLONTECH CO.) to clone cDNAs containing the entire coding region of the juvenile hormone acid methyltransferases from *Spodoptera litura* and *Helicoverpa armigera.*

For 3' RACE of the *Spodoptera litura* gene, the SL3R primer (SEQ ID NO: 21) was used to amplify the region from 366 to 994 of the entire nucleotide sequence, and for 5' RACE, the SL5R primer (SEQ ID NO: 22) was used to amplify the region from 1 to 393 of the entire nucleotide sequence. Both domains were laid overlapping to determine the entire nucleotide sequence. For 3' RACE of the *Helicoverpa armigera* gene, the HA3R primer (SEQ ID NO: 23) was used to amplify the region from 366 to 1193 of the entire nucleotide sequence, and for 5' RACE, the HA5R primer (SEQ ID NO: 24) was used to amplify the region from 1 to 391 of the entire nucleotide sequence, thus determining the nucleotide sequence.

SEQ ID NO: 8 describes the amino acid sequence of the juvenile hormone acid methyltransferase from *Spodoptera litura,* estimated from the cDNA sequence thus obtained (SEQ ID NO: 7) . The amino acid sequence identity with that of the juvenile hormone acid methyltransferase from *Bombyx mori* was 48%. SEQ ID NO: 10 describes the amino acid sequence of the juvenile hormone acid methyltransferase from *Helicoverpa armigera,* estimated from the obtained cDNA sequence (SEQ ID NO: 9). The amino acid sequence identity to that of the juvenile hormone acid methyltransferase of *Bombyx mori* was 54%.

cDNA synthesized by reverse transcription of the RNA extracted from the corpora allata of penultimate instar larva of *Spodoptera litura* was used as a template for PCR using the SLJF primer (SEQ ID NO: 25) and the SLJR primer (SEQ ID NO: 26), to amplify the coding region. The same method as in Example 2 was used to produce an expression vector, which was then used to produce a recombinant protein by the same method as in Example 3. The activity of the recombinant protein was determined by the same method as in Example 4 confirming that the protein encoded by the gene had juvenile hormone acid methyltransferase activity.

cDNA synthesized by reverse transcription of RNA extracted from the corpora allata of penultimate instar larva of *Helicoverpa armigera* was used as a template for PCR using the HAJF primer (SEQ ID NO: 27) and the HAJR primer (SEQ ID NO: 28) to amplify the coding region. The same method as in Example 2 was used to produce an expression vector, which was then used to produce a recombinant protein by the same method as in Example 3. The activity of the recombinant protein was determined by the same method as in Example 4, confirming that the protein encoded by the gene had juvenile hormone acid methyltransferase activity.

### (Example 7) Comparison of the primary structures of juvenile hormone acid methyltransferases

The present inventors compared the amino acid sequences of the juvenile hormone acid methyltransferases of *Drosophila melanogaster, Anopheles gambiae, Spodoptera litura,* and *Helicoverpa armigera* (Fig. 5). Seven motives, (1) to (7) below, were found to be conserved amino acid sequences. Of these, (2) coincided with the consensus sequence of methyltransferase (a portion of the S-adenosyl methionine binding sequence) (L(D/E)oGsGsG (SEQ ID NO: 29); wherein "o" is a hydrophobic amino acid, and "s" is a low molecular weight neutral amino acid; Gomi, T et al., Int. J. Biochem. 24:1639, 1992; Kagan, R.M. & Clarke, S., Arch. Biochem. Biophys. 310:417, 1994; Vidgren, J. et al., Science, 368:354, 1994), and was presumed to take part in the binding of S-adenosyl methionine. (1) 1 MNNAXLY(Q/E)(H/K/Q)ANSoQKRDA (SEQ ID NO: 30) (2) 38 (L/V/I)LD(L/V/I)GCGDG (SEQ ID NO: 31) (3) 62 (Q/R/K)L(L/V)GCDISE(Q/K)MV (SEQ ID NO: 32) (4) 104 FDHVFSFY(C/T/A)LHWV (SEQ ID NO: 33) (5) 145 P(V/I)FD(V/I/L)YR(I/V)L (SEQ ID NO: 34) (6) 165 DVE(K/R)YISPYHDS (SEQ ID NO: 35) (7) 263 YKL(I/V/L)VVYARK (SEQ ID NO: 36) The head numbers in (1) to (7) above are the number of amino acid residues in the juvenile hormone acid methyltransferases of *Bombyx mori* that correspond to each motif.

### Industrial Applicability

The present invention makes possible artificial regulation of juvenile hormone acid methyltransferase protein. This makes it possible to regulate juvenile hormone-controlled molting/metamorphosis, reproduction, diapause, blastogenesis, behavior, polymorphism, or life span of arthropods such as insects. Artificially regulating juvenile hormone acid methyltransferase protein enables pest control, and/or enhanced function of beneficial insects. Further, juvenile hormone acid methyltransferase protein can be used to produce or screen for compounds that bind to it, or compounds that regulate its activity or expression. Compounds obtained by screening can be used as very safe growth regulators that specifically act on insects.

## Claims

1. A DNA encoding a protein having a juvenile hormone acid methyltransferase activity, according to any one of (a) to (d) below:
(a) a DNA encoding a protein comprising an amino acid sequence of SEQ ID NO: 2, 4, 6, 8, or 10;
(b) a DNA comprising a coding region for a nucleotide sequence of SEQ ID NO: 1, 3, 5, 7, or 9;
(c) a DNA encoding a protein comprising an amino acid sequence of SEQ ID NO: 2, 4, 6, 8, or 10, wherein one or more amino acids are substituted, deleted, inserted, and/or added; or
(d) a DNA that hybridizes under stringent conditions with a DNA comprising a nucleotide sequence of SEQ ID NO: 1, 3, 5, 7, or 9.

2. A protein encoded by the DNA of Claim 1.

3. A DNA according to any one of (a) to (c) below:
(a) a DNA encoding an antisense RNA complementary to a transcription product of a DNA of Claim 1;
(b) a DNA encoding an RNA having a ribozyme activity which specifically cleaves a transcription product of a DNA of Claim 1; or
(c) a DNA encoding an RNA which inhibits the expression of a DNA of Claim 1 by an RNAi effect.

4. A vector inserted with the DNA of Claim 1 or 3.

5. An agent for regulating molting/metamorphosis, reproduction, diapause, blastogenesis, behavior, polymorphism, or life span of an arthropod, comprising as an active ingredient the DNA of Claim 1, or a vector inserted with said DNA.

6. The agent of Claim 5, wherein the arthropod is an insect.

7. The agent of Claim 6, wherein the regulatory agent is a reproductive maturation accelerating agent, a diapause terminating agent, or a life span shortening agent for an adult insect, a metamorphosis inhibiting agent for a larva and a pupa, or a diapause inducing agent for a larva.

8. The agent of Claim 6, wherein the regulatory agent is a pest control agent or a cocoon-promoting agent.

9. An agent for regulating molting/metamorphosis, reproduction, diapause, blastogenesis, behavior, polymorphism, or life span of an arthropod, comprising as an active ingredient the DNA of Claim 3, or a vector inserted with said DNA.

10. The agent of Claim 9, wherein the arthropod is an insect.

11. The agent of Claim 10, wherein the regulatory agent is a reproductive maturation inhibiting agent, a diapause inducing agent, or a life span elongating agent for an adult insect, a diapause inhibiting agent or a metamorphosis inducing agent for a larva.

12. The agent of Claim 10, wherein the regulatory agent is a pest control agent.

13. A transformed cell retaining the DNA of Claim 1 or 3, or a vector of Claim 4.

14. An individual transformed with the DNA of Claim 1 or 3, or a vector of Claim 4.

15. The individual of Claim 14, wherein the individual is an insect.

16. An antibody that binds to the protein of Claim 2.

17. The antibody of Claim 16, wherein the antibody is a monoclonal antibody.

18. An oligonucleotide comprising at least 15 nucleotides, wherein the oligonucleotide is complementary to the DNA of Claim 1 or its complementary strand.

19. A method of screening for a compound that binds to the protein of Claim 2, comprising steps (a) to (c) below:
(a) contacting a test compound with said protein;
(b) detecting the binding of the test compound and said protein; and
(c) selecting a compound which binds to said protein.

20. A method of screening for a compound that regulates the activity of the protein of Claim 2, comprising steps (a) to (c) below:
(a) contacting a test compound with said protein;
(b) determining the activity of said protein; and
(c) selecting a compound which increases or decreases the activity of said protein in comparison with a case where no test compound is administered.

21. A method of screening for a compound that regulates the expression level of a gene encoding the protein of Claim 2, comprising steps (a) to (d) below:
(a) providing a cell or cell extract comprising a DNA in which a reporter gene is functionally bound downstream of a promoter region of a gene encoding said protein;
(b) contacting a test compound with said cell or cell extract;
(c) determining the expression level of said reporter gene in said cell or cell extract; and
(d) selecting a compound which increases or decreases the expression level of said reporter gene in comparison with a case where no test compound is administered.

22. A method of screening for a compound that regulates the expression level of a gene encoding the protein of Claim 2, comprising steps (a) to (c) below:
(a) contacting a test compound with an insect individual or tissue culture;
(b) determining the expression level of the gene encoding the protein of Claim 2 in said insect individual or tissue culture; and
(c) selecting a compound which increases or decreases the expression level of said gene in comparison with a case where no test compound is administered.
